# EUROPEAN PATENT APPLICATION

(11) **EP 3 854 783 A1**
(43) Date of publication of application: **28.07.2021**
(21) Application number: 19862582.4
(22) Date of filing: 19.09.2019
(51) Int. Cl.: C07D 207/16, A61K 31/40, A61P 7/02, A61P 9/10, A61P 43/00

(54) **NOVEL SALT OF 4-(\{(4S)-1-(4-CARBAMIMIDOYLBENZOYL)-4-[4-(METHYLSULFONYL)PIPERAZIN-1-YL]-L-PROLYL\}AMINO)BENZOIC ACID, AND AND NOVEL CRYSTAL FORM OF SAID SALT**

(30) Priority: 20.09.2018 JP 2018176226
(71) Applicant: ONO Pharmaceutical Co., Ltd., Osaka-shi, Osaka 541-8526 (JP)
(72) Inventor: NISHIYAMA, Taihei, Mishima-gun, Osaka 618-8585 (JP); NEKADO, Takahiro, Mishima-gun, Osaka 618-8585 (JP)
(74) Representative: J A Kemp LLP
(86) International application number: PCT/JP2019/036806
(87) International publication number: WO 2020/059812

(57) **Abstract**

4-({(4S)-1-(4-Carbamimidoylbenzoyl)-4-[4-(methylsulfonyl)piperazin-1-yl]-L-prolyl}amino) benzoic acid di(4-toluenesulfonate) or dibenzenesulfonate or their crystal forms can be useful pharmaceutical ingredients because of their low hygroscopicity and excellent storage stability.

## Description

### TECHNICAL FIELD

The present invention relates to a novel salt form of 4-({(4S)-1-(4-carbamimidoylbenzoyl)-4-[4-(methylsulfonyl)piperazin-1-yl]-L-prolyl}amino) benzoic acid (hereinafter, may be abbreviated as Compound A.) and a novel crystal form thereof.

### BACKGROUND ART

Thromboembolism is a general term for thrombosis and thromboembolism which is a complication of thrombosis. Thromboembolism is ranked high along with cancer as the cause of death of adults, and has become important problems in recent years. Thromboembolism occurs by the formation of a thrombus at a site of vascular injury. Alternatively, thromboembolism occurs when a thrombus is released and is carried by the blood stream into another blood vessel where the thrombus obstructs the blood vessel. Thromboembolism includes, for example, venous thromboembolism which is a collective term for deep venous thrombosis and pulmonary embolism, cerebral stroke, angina pectoris, myocardial infarction, other various arterial and venous thrombosis and the like.

Tissue factor expressed on a vascular wall due to the injury of a blood vessel and the like becomes the starting point of the blood coagulation cascade and forms a complex with blood coagulation factor VII which is present in blood in a very small quantity. This complex activates blood coagulation factor IX and blood coagulation factor X, and activated blood coagulation factor X converts prothrombin to thrombin. Thrombin converts fibrinogen to fibrin and finally insoluble fibrin is formed (the initial stage). It is supposed that thrombin produced in the process promotes the formation of a thrombus at the initial stage and is important for hemostasis. On the other hand, it has been reported that thrombin activates blood coagulation factor XI and causes explosive thrombin production via activated blood coagulation factor XI (the amplification stage), which results in an increase in thrombi (see Non Patent Literatures 1 to 3).

For the treatment and/or prevention of thromboembolic disease, anticoagulant agents are generally used. Though conventional anticoagulant agents exhibit excellent antithrombotic actions, bleeding complications, which are serious side effects, have been problematic. Alternatively, in order not to cause bleeding complications, the doses of the agents are limited and it is supposed that there is a possibility that the agents do not exhibit sufficient antithrombotic actions. Under such conditions, an agent for treating and/or preventing thrombosis and thromboembolism having a novel mechanism of action, which suppresses the growth of or increase in pathological thrombi and does not affect the formation of hemostatic thrombi, is required. As one of the targets of the agent, blood coagulation factor XIa is attracting attention in recent years. Blood coagulation factor XI is one of plasma serine proteases which are involved in the regulation of blood coagulation and becomes active-type blood coagulation factor XIa by activated blood coagulation factor XII, thrombin or itself. Blood coagulation factor XIa is one of constituents of the blood coagulation pathway which is referred to as the intrinsic system or the contact system in the classical blood coagulation cascade and activates blood coagulation factor IX by selectively cleaving peptide bonds of Arg-Ala and Arg-Val. The safety of blood coagulation factor XIa is supported by the observations that the blood coagulation XI deficiency in humans, which is called hemophilia C, results in mild to moderate bleeding characterized primarily by postoperative or posttraumatic hemorrhage. In addition, the effects and the high safety of blood coagulation factor XIa are demonstrated by the experimental results of experimental thrombosis and bleeding models which used blood coagulation XI deficient mice and the experimental results of an anti-blood coagulation XI neutralizing antibody or an antisense in experimental thrombosis and bleeding models which used monkeys or rabbits, in addition to the results of observations of the blood coagulation factor XIa deficiency in humans (see Non Patent Literatures 4 to 8).

Based on the above results, it is expected that blood coagulation factor XIa is a very attractive target without exhibiting the side effect of bleeding when developing an antithrombotic agent for treatment and/or prevention and a blood coagulation factor XIa inhibitor becomes a very potent and safe antithrombotic agent for treatment or prevention without having any undesirable side effects such as bleeding.

Incidentally, Patent Literatures 1 and 2 describe that a compound group containing a trifluoroacetate of Compound A is a selective blood coagulation factor XIa inhibitor and is useful as a therapeutic drug for thromboembolic diseases and the like.

### CITATIONS LISTS

### Patent Literatures

Patent Literature 1: WO 2013/174937 A
Patent Literature 2: JP 2015-120685 A

### Non Patent Literatures

Non Patent Literature 1: Blood Coagulation and Fibrinolysis, 2006, Vol. 17, pages 251-257
Non Patent Literature 2: Science, 1991, Vol. 253, pages 909-912
Non Patent Literature 3: Blood, 2003, Vol. 102, pages 953-955
Non Patent Literature 4: Journal of Thrombosis and Haemostasis, 2005, Vol. 3, pages 695-702
Non Patent Literature 5: Journal of Thrombosis and Haemostasis, 2006, Vol. 4, pages 1982-1988
Non Patent Literature 6: Blood, 2012, Vol. 119, pages 2401-2408
Non Patent Literature 7: Blood, 2009, Vol. 113, pages 936-944
Non Patent Literature 8: Journal of Thrombosis and Haemostasis, 2006, Vol. 4, pages 1496-1501

### SUMMARY OF INVENTION

### TECHNICAL PROBLEMS

Since the trifluoroacetate of Compound A described in Patent Literatures 1 and 2 was amorphous, it was desired to acquire a new crystal salt or the like which is excellent in handling as pharmaceuticals. Therefore, an object of the present invention is to provide a salt that forms a crystal and is excellent in low hygroscopicity and/or storage stability among various acid addition salts of Compound A.

### SOLUTIONS TO PROBLEMS

The present inventors have conducted studies to solve the above problems and have found that pentahydrate and monoacetate form crystals. However, since these crystals had problems in storage stability and hygroscopicity, further studies were conducted. As a result, the present inventors have found that ditosylate and dibesylate of Compound A formed a crystal, and the crystals are excellent in low moisture absorption and storage stability, and have completed the present invention.

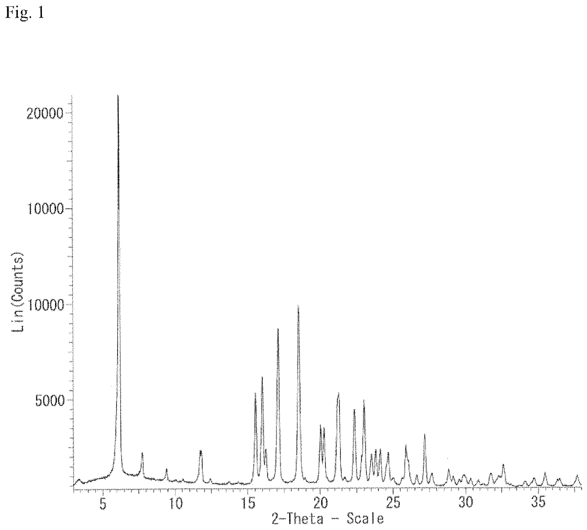

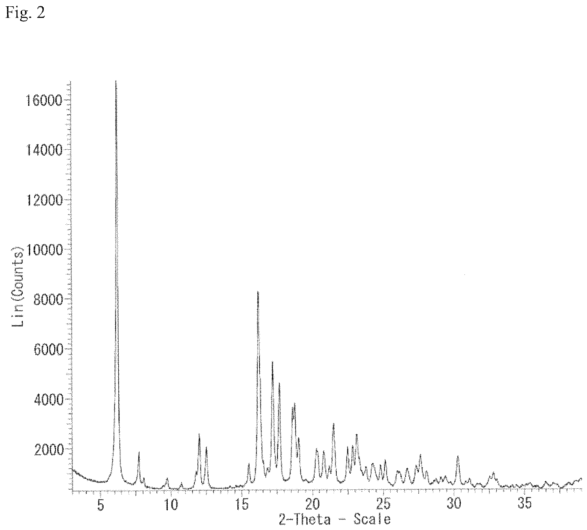

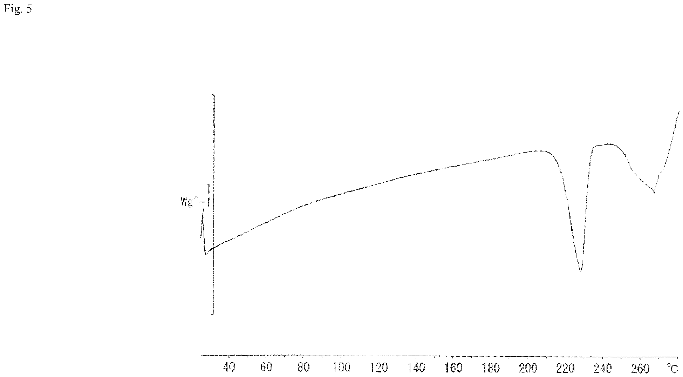

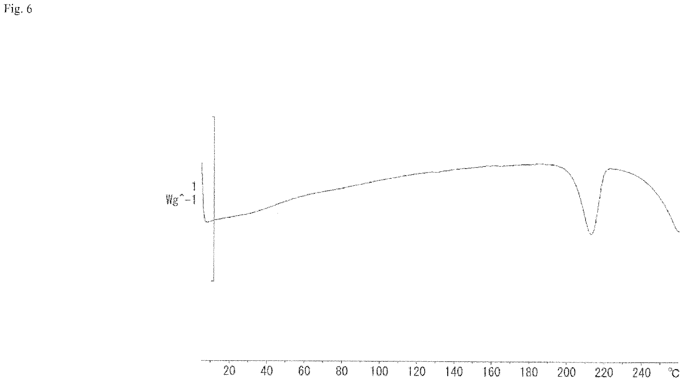

The present invention provides, for example, the following embodiments.
[1] 4-({(4S)-1-(4-Carbamimidoylbenzoyl)-4-[4-(methylsulfonyl)piperazin-1-yl]-L-prolyl}amino) benzoic acid di(4-toluenesulfonate);
[2] A crystal of the salt according to [1] above (The salt according to [1] above, which is in a crystal form);
[3] The crystal according to [2] above, having, in a powder X-ray diffraction spectrum, at least two or more diffraction peaks at diffraction angles (20) selected from about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees;
[4] The crystal according to [2] above, having, in a powder X-ray diffraction spectrum, at least three or more diffraction peaks at diffraction angles (2θ) selected from about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees;
[4-1] The crystal according to [2] above, having, in a powder X-ray diffraction spectrum, at least four or more diffraction peaks at diffraction angles (2θ) selected from about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees;
[4-2] The crystal according to [2] above, having, in a powder X-ray diffraction spectrum, at least five or more diffraction peaks at diffraction angles (2θ) selected from about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees;
[4-3] The crystal according to [2] above, having, in a powder X-ray diffraction spectrum, at least one, two, three, four or five or more diffraction peaks at diffraction angles (20) selected from about 6.2, about 15.6, about 16.0, about 17.1, about 18.6, and about 22.4 degrees;
[5] The crystal according to [2] above, having, in a powder X-ray diffraction spectrum, diffraction peaks at diffraction angles (2θ) of about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees;
[6] The crystal according to [2] above, characterized by a powder X-ray diffraction spectrum chart shown in Fig. 1;
[7] The crystal according to any one of [2], [3], [4], [4-1], [4-2], [4-3], [5] and [6] above, having an onset temperature of about 216°C or an endothermic peak temperature of about 228°C in differential scanning calorimetry;
[8] The crystal according to [7] above, characterized by a differential scanning calorimetry chart shown in Fig. 5;
[9] 4-({(4S)-1-(4-Carbamimidoylbenzoyl)-4-[4-(methylsulfonyl)piperazin-1-yl]-L-prolyl}amino) benzoic acid dibenzenesulfonate;
[10] A crystal of the salt according to [9] above (The salt according to [9] above, which is in a crystal form);
[11] The crystal according to [10] above, having, in a powder X-ray diffraction spectrum, at least two or more diffraction peaks at diffraction angles (20) selected from about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees;
[12] The crystal according to [10] above, having, in a powder X-ray diffraction spectrum, at least three or more diffraction peaks at diffraction angles (2θ) selected from about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees;
[12-1] The crystal according to [10] above, having, in a powder X-ray diffraction spectrum, at least four or more diffraction peaks at diffraction angles (2θ) selected from about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees;
[12-2] The crystal according to [10] above, having, in a powder X-ray diffraction spectrum, at least five or more diffraction peaks at diffraction angles (20) selected from about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees;
[12-3] The crystal according to [10] above, having, in a powder X-ray diffraction spectrum, at least one, two, three, four or five or more diffraction peaks at diffraction angles (2θ) selected from about 6.2, about 12.5, about 16.2, about 17.2, about 17.7 and about 21.5 degrees;
[13] The crystal according to [10] above, having, in a powder X-ray diffraction spectrum, diffraction peaks at diffraction angles (20) of about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees;
[14] The crystal according to [10] above, characterized by a powder X-ray diffraction spectrum chart shown in Fig. 2;
[15] The crystal according to any one of [10], [11], [12], [12-1], [12-2], [12-3], [13] and [14] above, having an onset temperature of about 203°C or an endothermic peak temperature of about 213°C in differential scanning calorimetry;
[16] The crystal according to [15] above, characterized by a differential scanning calorimetry chart shown in Fig. 6;
[17] A pharmaceutical composition containing 4-(1(4S)-1-(4-carbamimidoylbenzoyl)-4-[4-(methylsulfonyl)piperazin-1-yl]-L-prolyl}amino) benzoic acid di(4-toluenesulfonate) or the crystal according to any one of [2] to [4], [4-1] to [4-3] and [5] to [8] above (A pharmaceutical composition containing the 4-({(4S)-1-(4-carbamimidoylbenzoyl)-4-[4-(methylsulfonyl)piperazin-1-yl]-L-prolyl}amino) benzoic acid di(4-toluenesulfonate) according to [1] to [4], [4-1] to [4-3] and [5] to [8] above and a pharmaceutically acceptable carrier);
[18] A pharmaceutical composition containing 4-({(4S)-1-(4-carbamimidoylbenzoyl)-4-[4-(methylsulfonyl)piperazin-1-yl]-L-prolyl} amino) benzoic acid dibenzenesulfonate or the crystal according to any one of [10] to [12], [12-1] to [12-3] and [13] to [16] above (A pharmaceutical composition containing the 4-({(4S)-1-(4-carbamimidoylbenzoyl)-4-[4-(methylsulfonyl)piperazin-1-yl]-L-prolyl}amino) benzoic acid dibenzenesulfonate according to [9] to [12], [12-1] to [12-3] and [13] to [16] above and a pharmaceutically acceptable carrier);
[19] The pharmaceutical composition according to [17] or [18] above, which is a blood coagulation factor Xla inhibitor;
[20] The pharmaceutical composition according to [17] or [18] above, which is a drug for preventing and/or treating blood coagulation factor Xla-related disease;
[21] The pharmaceutical composition according to [20] above, wherein the blood coagulation factor XIa-related disease is thromboembolic disease;
[22] The pharmaceutical composition according to [21] above, wherein the thromboembolic disease is acute coronary syndrome, disseminated intravascular coagulation (DIC) or cerebral infarction;
[23] An agent for preventing and/or treating thromboembolic disease, containing 4-({(4S)-1-(4-carbamimidoylbenzoyl)-4-[4-(methylsulfonyl)piperazin-1-yl]-L-prolyl}amino) benzoic acid di(4-toluenesulfonate) or the crystal according to any one of [2] to [4], [4-1] to [4-3] and [5] to [8] above;
[24] A method for preventing and/or treating thromboembolic disease, including administering to an mammal an effective amount of 4-({(4S)-1-(4-carbamimidoylbenzoyl)-4-[4-(methylsulfonyl)piperazin-1-yl]-L-prolyl}amino) benzoic acid di(4-toluenesulfonate) or the crystal according to any one of [2] to [4], [4-1] to [4-3] and [5] to [8] above;
[25] 4-({(4S)-1-(4-Carbamimidoylbenzoyl)-4-[4-(methylsulfonyl)piperazin-1-yl]-L-prolyl}amino) benzoic acid di(4-toluenesulfonate) or the crystal according to any one of [2] to [4], [4-1] to [4-3] and [5] to [8] above, which is used for prevention and/or treatment of thromboembolic disease;
[26] A use of 4-({(4S)-1-(4-carbamimidoylbenzoyl)-4-[4-(methylsulfonyl)piperazin-1-yl]-L-prolyl} amino) benzoic acid di(4-toluenesulfonate) or the crystal according to any one of [2] to [4], [4-1] to [4-3] and [5] to [8] above, for production of an agent for preventing and/or treating thromboembolic disease;
[27] An agent for preventing and/or treating thromboembolic disease, containing 4-({(4S)-1-(4-carbamimidoylbenzoyl)-4-[4-(methylsulfonyl)piperazin-1-yl]-L-prolyl}amino) benzoic acid dibenzenesulfonate or the crystal according to any one of [10] to [12], [12-1] to [12-3] and [13] to [16] above;
[28] A method for preventing and/or treating thromboembolic disease, including administering to an mammal an effective amount of 4-({(4S)-1-(4-carbamimidoylbenzoyl)-4-[4-(methylsulfonyl)piperazin-1-yl]-L-prolyl}amino) benzoic acid dibenzenesulfonate or the crystal according to any one of [10] to [12], [12-1] to [12-3] and [13] to [16] above;
[29] 4-({(4S)-1-(4-Carbamimidoylbenzoyl)-4-[4-(methylsulfonyl)piperazin-1-yl]-L-prolyl}amino) benzoic acid dibenzenesulfonate or the crystal according to any one of [10] to [12], [12-1] to [12-3] and [13] to [16] above, which is used for prevention and/or treatment of thromboembolic disease;
[30] A use of 4-({(4S)-1-(4-carbamimidoylbenzoyl)-4-[4-(methylsulfonyl)piperazin-1-yl]-L-prolyl}amino) benzoic acid dibenzenesulfonate or the crystal according to any one of [10] to [12], [12-1] to [12-3] and [13] to [16] above, for production of an agent for preventing and/or treating thromboembolic disease;
[31] A method for producing the crystal according to [2] above, including using tetrahydrofuran in a production process;
[32] A method for producing the crystal according to [10] above, including using tetrahydrofuran in a production process.

### ADVANTAGEOUS EFFECTS OF INVENTION

Ditosylate and dibesylate of Compound A form crystals and are useful as pharmaceutical ingredients with low hygroscopicity and excellent storage stability.

### BRIEF DESCRIPTION OF DRAWINGS

Fig. 1 represents a powder X-ray diffraction spectrum chart of ditosylate of Compound A (vertical axis represents intensity (counts), and horizontal axis represents 2θ (degrees)).
Fig. 2 represents a powder X-ray diffraction spectrum chart of dibesylate of Compound A (vertical axis represents intensity (counts), and horizontal axis represents 2θ (degrees)).
Fig. 3 represents a powder X-ray diffraction spectrum chart of monoacetate of Compound A (vertical axis represents intensity (counts), and horizontal axis represents 2θ (degrees)).
Fig. 4 represents a powder X-ray diffraction spectrum chart of pentahydrate of Compound A (vertical axis represents intensity (counts), and horizontal axis represents 2θ (degrees)).
Fig. 5 represents a differential scanning calorimetry (DSC) chart of ditosylate of Compound A (vertical axis represents heat flux (W/g), and horizontal axis represents temperature (°C)).
Fig. 6 represents a differential scanning calorimetry (DSC) chart of dibesylate of Compound A (vertical axis represents heat flux (W/g), and horizontal axis represents temperature (°C)).
Fig. 7 represents a differential scanning calorimetry (DSC) chart of monoacetate of Compound A (vertical axis represents heat flux (W/g), and horizontal axis represents temperature (°C)).
Fig. 8 represents a differential scanning calorimetry (DSC) chart of pentahydrate of Compound A (vertical axis represents heat flux (W/g), and horizontal axis represents temperature (°C)).
Fig. 9 represents an isotherm adsorption curve of ditosylate of Compound A (vertical axis represents change in dry mass (%), and horizontal axis represents relative humidity (RH) (%)).
Fig. 10 represents an isotherm adsorption curve for dibesylate of Compound A (vertical axis represents change in dry mass (%), and horizontal axis represents relative humidity (RH) (%)).
Fig. 11 represents an isotherm adsorption curve for monoacetate of Compound A (vertical axis represents change in dry mass (%), and horizontal axis represents relative humidity (RH) (%)).
Fig. 12 represents an isotherm adsorption curve for pentahydrate of Compound A (vertical axis represents change in dry mass (%), and horizontal axis represents relative humidity (RH) (%)).
Fig. 13 represents a powder X-ray diffraction spectrum chart of monoacetate of Compound A after storage at 25°C and 60% RH for 2 months (vertical axis represents intensity (counts), and horizontal axis represents 2θ (degrees)).
Fig. 14 represents a powder X-ray diffraction spectrum chart of ditosylate of Compound A (vertical axis represents intensity (counts), and horizontal axis represents 20 (degrees)).
Fig. 15 represents a powder X-ray diffraction spectrum chart of ditosylate of Compound A after storage at 25°C and 60% RH for 2 months (vertical axis represents intensity (counts), and horizontal axis represents 2θ (degrees)).

### DESCRIPTION OF EMBODIMENTS

The present invention will be described in details hereinbelow.

In the present invention, 4-({(4S)-1-(4-carbamimidoylbenzoyl)-4-[4-(methylsulfonyl)piperazin-1-yl]-L-prolyl}amino) benzoic acid (Compound A) means a compound represented by the following structural formula: 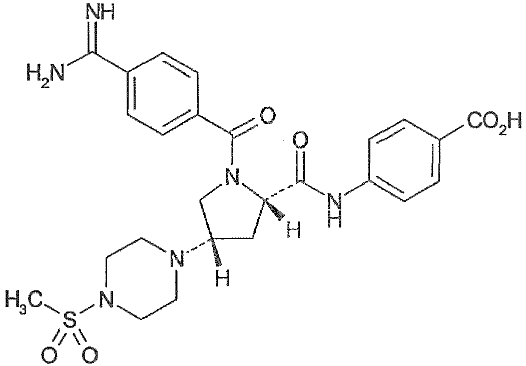 wherein a symbol 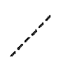

represents α-configuration, and a symbol 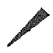

represents β-configuration.

The compound represented by the above structural formula can also be named 4-[({(2S,4S)-1-(4-carbamimidoylbenzoyl)-4-[4-(methylsulfonyl)-1-piperazinyl]-2-pyrrolidinyl}carbonyl)amino] benzoic acid.

A crystal of ditosylate of Compound A is characterized by physicochemical data of at least one of the following (a) and (b). Preferably, it is characterized by the physicochemical data of both (a) and (b). (a) A powder X-ray diffraction spectrum having substantially the same diffraction angles (2θ) as diffraction angles (20) shown in Fig. 1 or diffraction angles (2θ) shown in Table 2, or having at least one, two, three, four, five, or more than five peaks at diffraction angles (20) selected from substantially the same diffraction angles (20) as the diffraction angles (20) shown in Table 2, b) having differential scanning calorimetry (DSC) as shown in Fig. 5, or an endothermic peak with an onset temperature of about 216°C or an endothermic peak temperature of about 228°C.

One embodiment of the crystal of ditosylate of Compound A is a crystal having, in a powder X-ray diffraction spectrum, at least one, two, three, four or five or more peaks at diffraction angles (2θ) selected from about 6.2, about 15.6, about 16.0, about 17.1, about 18.6 and about 22.4 degrees, and another embodiment is a crystal having, in a powder X-ray diffraction spectrum, diffraction angle (2θ) peaks at about 6.2, about 15.6, about 16.0, about 17.1, about 18.6 and about 22.4 degrees. Yet another embodiment of the crystal of ditosylate of Compound A is a crystal having at least one, two, three, four or five or more peaks at diffraction angles (20) selected from about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9 and about 27.2 degrees, and yet another embodiment is a crystal having peaks at about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9 and about 27.2 degrees.

A crystal of dibesylate of Compound A is characterized by physicochemical data of at least one of the following (c) and (d). Preferably, it is characterized by the physicochemical data of both (c) and (d). (c) A powder X-ray diffraction spectrum having substantially the same diffraction angles (20) as diffraction angles (20) shown in Fig. 2 or diffraction angles (20) shown in Table 3 below, or having at least one, two, three, four, five, or more than five peaks at diffraction angles (20) selected from substantially the same diffraction angles (2θ) as the diffraction angles (20) shown in Table 3, d) having differential scanning calorimetry (DSC) as shown in Fig. 6 below, or an endothermic peak with an onset temperature of about 203°C or an endothermic peak temperature of about 213°C.

One embodiment of the crystal of dibesylate of Compound A is a crystal having, in a powder X-ray diffraction spectrum, at least one, two, three, four or five or more peaks at diffraction angles (2θ) selected from about 6.2, about 12.5, about 16.2, about 17.2, about 17.7 and about 21.5 degrees, and another embodiment is a crystal having, in a powder X-ray diffraction spectrum, diffraction angle (20) peaks at about 6.2, about 12.5, about 16.2, about 17.2, about 17.7 and about 21.5 degrees. Yet another embodiment of the crystal of dibesylate of Compound A is a crystal having at least one, two, three, four or five or more peaks at diffraction angles (20) selected from about 6.2, about 7.7, about 12.0, about 12.5, about 16.2, about 17.2, about 17.7, about 18.6, about 18.8, about 19.0, about 20.3, about 20.8, about 21.5, about 22.5, about 22.9 and about 23.1 degrees, and yet another embodiment is a crystal having peaks at about 6.2, about 7.7, about 12.0, about 12.5, about 16.2, about 17.2, about 17.7, about 18.6, about 18.8, about 19.0, about 20.3, about 20.8, about 21.5, about 22.5, about 22.9 and about 23.1 degrees.

### [Isotope labeling]

The ditosylate and dibesylate of Compound A may have one or more atoms replaced by isotopes, and isotope-labeled Compound A, for example, ditosylate and dibesylate of Compound A incorporating a radioisotope are useful in studies of tissue distribution of drugs and/or substrates. Examples of the isotope include ²H, ³H, ¹¹C, ¹³C, ¹⁴C, ¹³N, ¹⁵N, ¹⁵O, ¹⁷O, ¹⁸O, ³⁵S, ¹⁸F, ³⁶Cl, ¹²³I, ¹²⁵I, and the like.

### [Toxicity]

Since toxicity of ditosylate and dibesylate of Compound A is low, they can be used safely.

### [Application to pharmaceuticals]

Since the ditosylate and dibesylate of Compound A have blood coagulation factor XIa (factor XIa) inhibitory activity, they can be used as agents for preventing and/or treating factor XIa-related disease, for example, thromboembolic disease, in mammals, especially humans.

Examples of the thromboembolic disease include thromboembolic diseases such as arterial cardiovascular thromboembolic disease, venous cardiovascular thromboembolic disease, arterial cerebrovascular thromboembolic disease, venous cerebrovascular thromboembolic disease, and thromboembolic diseases in the atrium or peripheral circulation.

Examples of the arterial cardiovascular thromboembolic disease include coronary artery disease, ischemic cardiomyopathy, acute coronary syndrome, coronary artery thrombosis, ischemic complications of unstable angina and non-Q wave myocardial infraction, ST-segment elevation and/or non ST-segment elevation acute myocardial infarction which is medically cared or involves percutaneous coronary intervention, angina pectoris such as stable (exercise-induced) angina pectoris, variant angina pectoris, unstable angina, myocardial infarction (such as first myocardial infarction and recurrent myocardial infarction), acute myocardial infarction, reocclusion and stenosis of a blood vessel after coronary artery bypass graft surgery, reocclusion and stenosis after percutaneous transluminal angioplasty, cardiac/transcoronary stent implantation and after thrombolytic therapy for coronary artery, ischemic sudden death and the like.

Examples of the venous cardiovascular thromboembolic disease include deep venous thrombosis (DVT) and/or pulmonary embolism (PE) in major general surgery, abdominal surgery, hip replacement arthroplasty, knee replacement arthroplasty, hip fracture surgery, multiple bone fracture, multiple trauma, traumatic injury, spinal cord injury, burn injury or at the time of entering critical care unit, DVT and/or PE in a patient with acute disease with a significantly limited physical activity, DVT and/or PE in a patient receiving cancer chemotherapy, DVT and/or PE in a patient with cerebral stroke, symptomatic or asymptomatic DVT regardless of the presence/absence of PE and the like.

Examples of the arterial cerebrovascular thromboembolic disease include cerebral stroke, ischemic stroke, the acute phase of cerebral infarction, cerebral stroke in a patient with nonvalvular atrial fibrillation or valvular atrial fibrillation, cerebral arterial thrombosis, cerebral infarction, transient ischemic attack (TIA), lacunar infarct, atherothrombotic cerebral infarction, cerebral arterial embolism, cerebral thrombosis, cerebrovascular disorder, asymptomatic cerebral infarction and the like.

Examples of the venous cerebrovascular thromboembolic disease include intracranial venous thrombosis, cerebral embolism, cerebral thrombosis, venous sinus thrombosis, intracranial venous sinus thrombosis, cavernous sinus thrombosis and the like.

Examples of the thromboembolic disease in the atrium or peripheral circulation include venous thrombosis, systemic venous thromboembolism, thrombophlebitis, nonvalvular and valvular atrial fibrillation, cardiogenic embolism, disseminated intravascular coagulation (DIC), sepsis, acute respiratory distress syndrome (ARDS), acute lung injury (ALI), antiphospholipid antibody syndrome, renal embolism, atherosclerosis, atherothrombosis, peripheral arterial occlusive disease (PAOD), peripheral arterial disease, arterial embolism, thrombosis induced by treatment exposed to a surface of a medical implant, a device, or an artifact which promotes thrombosis (such as a catheter, a stent, a prosthetic cardiac valve or a hemodialyzer), and the like.

Preferable examples of the thromboembolic disease unstable angina, acute coronary syndrome, atrial fibrillation, myocardial infarction (such as first myocardial infarction and recurrent myocardial infarction), ischemic sudden death, transient ischemic attack, cerebral stroke, atherosclerosis, peripheral occlusive arterial disease, venous thrombosis, deep venous thrombosis, thrombophlebitis, arterial embolism, coronary artery thrombosis, cerebral arterial thrombosis, cerebral embolism, kidney embolism, portal vein thrombosis, pulmonary embolism, pulmonary infarction, liver embolism, disseminated intravascular coagulation (DIC), sepsis, acute respiratory distress syndrome (ARDS), acute lung injury (ALI), antiphospholipid antibody syndrome, thrombosis due to coronary artery bypass graft surgery, thrombosis induced by treatment exposed to a surface of a medical implant, a device, or an artifact which promotes thrombosis (such as a catheter, a stent, a prosthetic cardiac valve or a hemodialyzer), and the like (for example, blood coagulation when using hemodialysis, artificial heart-lung machine or other extracorporeal circulation devices).

When the ditosylate or dibesylate of Compound A is applied to a pharmaceutical, the ditosylate or dibesylate of Compound A may be used not only as a single agent, but also as a combined medicine by being combined with other active ingredient(s), for example, agent(s) and the like which are listed hereinbelow for the purpose, for example, of:
(1) complementation and/or enhancement of the effects of preventing, treating and/or ameliorating symptoms,
(2) improvement in the kinetics or absorption, and reduction of the dose, and/or
(3) reduction of the side effects.

When the ditosylate or dibesylate of Compound A is used for preventing and/or treating thromboembolic disease, examples of combined agent(s) which is used in combination with the ditosylate or dibesylate of Compound A include an anticoagulant agent, an antiplatelet agent, a thrombolytic agent, a fibrinolytic agent, a serine protease inhibitor, an elastase inhibitor, a steroid, a combination thereof and the like.

Examples of the anticoagulant agent include a thrombin inhibitor, an antithrombin III activator, a heparin cofactor II activator, other factor XIa inhibitors, a plasma and/or tissue kallikrein inhibitor, an inhibitor of plasminogen activator inhibitor (PAI-1), an inhibitor of thrombin-activatable fibrinolysis inhibitor (TAFI), a factor VIIa inhibitor, a factor VIIIa inhibitor, a factor IXa inhibitor, a factor Xa inhibitor, a factor XIIa inhibitor, a combination thereof and the like.

Examples of the antiplatelet agent include a GPII/IIIa blocker, a protease-activated receptor (PAR-1) antagonist, a PAR-4 antagonist, a phosphodiesterase III inhibitor, other phosphodiesterase inhibitors, a P2X1 antagonist, a P2Y1 receptor antagonist, a P2Y12 antagonist, a thromboxane receptor antagonist, a thromboxane A2 synthetase inhibitor, a cyclooxygenase-1 inhibitor, a phospholipase D1 inhibitor, a phospholipase D2 inhibitor, a phospholipase D inhibitor, a glycoprotein VI (GPVI) antagonist, a glycoprotein Ib (GPIB) antagonist, a GAS6 antagonist, aspirin, a combination thereof and the like.

Preferably, the combined agent is an antiplatelet agent.

Preferable examples of the antiplatelet agent include clopidogrel, prasugrel, ticagrelor, cangrelor, elinogrel, cilostazol, sarpogrelate, iloprost, beraprost, limaprost and/or aspirin, a combination thereof and the like.

Preferably, the combined agent is warfarin, unfractionated heparin, low-molecular-weight heparin, enoxaparin, dalteparin, bemiparin, tinzaparin, semuloparin sodium (AVE-5026), danaparoid, a synthesized pentasaccharide, fondaparinux, hirudin, disulfatohirudin, lepirudin, bivalirudin, desirudin, argatroban, aspirin, ibuprofen, naproxen, sulindac, indomethacin, mefenamate, droxicam, diclofenac, sulfinpyrazone, piroxicam, ticlopidine, clopidogrel, prasugrel, ticagrelor, cangrelor, elinogrel, cilostazol, sarpogrelate, iloprost, beraprost, limaprost, tirofiban, eptifibatide, abciximab, mclagatran, ximelagatran, dabigatran, rivaroxaban, apixaban, edoxaban, darexaban, betrixaban, TAK-442, tissue plasminogen activator, a modified tissue plasminogen activator, anistreplase, urokinase, streptokinase, gabexate, gabexate mesilate, nafamostat, sivelestat, sivelestat sodium hydrate, alvelestat (AZD-9668), ZD-8321/0892, ICI-200880, human elafin (tiprelestat), elafin, α1-antitrypsin (A1AT), cortisone, betamethasone, dexamethasone, hydrocortisone, methylprednisolone, prednisolone, triamcinolone or a combination thereof.

In another embodiment, examples of the combined agent in the present invention include a potassium channel opener, a potassium channel blocker, a calcium channel blocker, an inhibitor of sodium-hydrogen exchanger, an antiarrhythmic agent, an antiarteriosclerotic agent, an anticoagulant agent, an antiplatelet agent, an antithrombotic agent, a thrombolytic agent, a fibrinogen antagonist, an antihypertensive diuretic, an ATPase inhibitor, a mineralocorticoid receptor antagonist, a phosphodiesterase inhibitor, an antidiabetic agent, a protease inhibitor, an elastase inhibitor, an anti-inflammatory agent, an antioxidant, an angiogenesis-modulating agent, an agent for treating osteoporosis, hormone replacement therapy, a hormone receptor-modulating agent, an oral contraceptive, an anti-obesity drug, an antidepressant drug, an antianxiety agent, an antipsychotic agent, an antiproliferative agent, an antitumor agent, antiulcer and antigastroesophageal reflux agents, a growth hormone agent and/or a growth hormone secretagogue, a thyroid-mimetic, an anti-infective agent, an antiviral agent, an antimicrobial agent, an antifungal agent, a drug for treating hypercholesterolemia/dyslipidemia and therapy for improving lipid profile, preconditioning of simulated ischemia and/or an agent for stunned myocardium, a combination thereof and the like.

In a preferable embodiment, examples of the combined agent in the present invention include an antiplatelet agent and a combination thereof.

In another embodiment, examples of the combined agent in the present invention further include an antiarrhythmic agent, an antihypertensive agent, an anticoagulant agent, an antiplatelet agent, a thrombolytic agent, a fibrinolytic agent, a calcium channel blocker, a potassium channel blocker, a cholesterol/lipid-lowering agent, a serine protease inhibitor, an elastase inhibitor, an anti-inflammatory agent, a combination thereof and the like.

Examples of the antiarrhythmic agent include an IKur inhibitor, an elastase inhibitor, a serine protease inhibitor, a steroid and the like.

Examples of the antihypertensive agent include an ACE inhibitor, an AT-1 receptor antagonist, a β-adrenergic receptor antagonist, an ETA receptor antagonist, a dual ETA/AT-1 receptor antagonist, a vasopeptidase inhibitor and the like.

In a preferable embodiment, examples of the combined agent in the present invention include an antiplatelet agent and a combination thereof.

The combined medicine of the ditosylate or dibesylate of Compound A with the above-described other agent(s) may be administered in the form of a compounding agent in which both ingredients are compounded in a preparation or may be administered in the form of separate preparations by the same route of administration or different routes of administration. When the separate preparations are administered, the preparations are not necessarily administered concomitantly, but as needed, each of the preparations may be administered with a time difference. In addition, in the case of the administrations with a time difference, the order of administrations is not particularly limited, but may be appropriately adjusted in order to achieve the desired drug efficacy.

The dose of the above-described other agent(s) which is used in combination with the ditosylate or dibesylate of Compound A can be appropriately increased or decreased based on the clinically used dose of the agent(s) or an agent similar thereto. In addition, the compounded ratio of the ditosylate or dibesylate of Compound A and other agent(s) can be appropriately adjusted by considering the age and body weight of the subject of administration, the method for administration, the duration of administration, the target disease, the symptom and the like. Approximately 0.01 to 100 parts by weight of other agent(s) may be combined with 1 part by weight of the ditosylate or dibesylate of Compound A. Two or more kinds of other agent(s) may be used. In addition, examples of the other agent(s) include not only those listed above, but also drug(s) having the same mechanism as those listed above. The drug(s) having the same mechanism as those listed above includes not only those which have been found up to now but also those which will be found in future.

The dose of ditosylate or dibesylate of Compound A varies depending on the age, the body weight, the symptom, the therapeutic effect, the method for administration, the duration of the treatment and the like. However, normally, the dose per adult is in the range of from 1 mg to 300 mg per administration, from one to several oral administrations per day or the dose per adult is in the range of from 0.1 mg to 30 mg per administration, from one to several parenteral administrations per day. Alternatively, the dose is continuously administrated intravenously for a period of time in the range of 1 to 24 hours per day.

Of course, the dose varies depending on various factors as described above, and therefore, there are some cases in which a dose below the above-described dose is sufficient and there are other cases in which administration of a dose which exceeds the above-described range is required.

In order to use the ditosylate or dibesylate of Compound A as a single agent or in combination with another agent as a concomitant drug, for the purpose of prevention and/or treatment of the above diseases, the substance as an active ingredient is usually formulated with various additives or a pharmaceutically acceptable carrier such as a solvent and then administered systemically or topically in oral or parenteral form. Here, the pharmaceutically acceptable carrier means a substance other than the active ingredient, which is generally used in the preparation of pharmaceuticals. The pharmaceutically acceptable carrier preferably has no pharmacological action at a dose of the preparation, is harmless, and does not interfere with the therapeutic effect of the active ingredient. In addition, the pharmaceutically acceptable carrier can also be used for the purpose of enhancing usefulness of the active ingredient and the preparation, facilitating formulation, stabilizing quality, improving usability, and the like. Specifically, substances such as those described in "Encyclopedia of Pharmaceutical Additives" published by Yakuji Nippo, Limited in 2000 (edited by International Pharmaceutical Excipients Council Japan) or the like may be appropriately selected according to the purpose.

The ditosylate or dibesylate of Compound A is normally administered systemically or topically, in the form of an oral preparation or a parenteral preparation. Examples of the oral preparation include an oral liquid preparation (such as an elixir, a syrup, a pharmaceutically acceptable liquid agent, a suspension and an emulsion), an oral solid preparation (such as a tablet (including a sublingual tablet and an orally disintegrating tablet), a pill, a capsule (including a hard capsule, a soft capsule, a gelatin capsule and a microcapsule), a powdered agent, a granule and a lozenge) and the like. Examples of the parenteral preparation include a liquid preparation (such as an injection preparation (such as an intravitreal injection preparation, a subcutaneous injection preparation, an intravenous injection preparation, an intramuscular injection preparation, an intraperitoneal injection preparation and a preparation for drip infusion), an eye drop (such as an aqueous eye drop (such as an aqueous ophthalmic solution, an aqueous ophthalmic suspension, a viscous eye drop and a solubilized eye drop) and a non-aqueous eye drop (such as a non-aqueous ophthalmic solution and a non-aqueous ophthalmic suspension))), an external preparation (such as an ointment (such as an ophthalmic ointment)), an ear drop and the like. The above-described preparation may be a controlled-release preparation such as an immediate-release preparation and a sustained release preparation. The above-described preparation can be prepared by a known method, for example, by a method described in Pharmacopeia of Japan or the like.

The oral liquid preparation as an oral preparation is prepared, for example, by dissolving, suspending or emulsifying an active ingredient in a generally used diluent (such as purified water, ethanol and a mixed liquid thereof), In addition, the liquid preparation may further contain a wetting agent, a suspending agent, an emulsifying agent, a sweetening agent, a flavoring agent, a perfume, a preservative, a buffer agent and the like.

The oral solid preparation as an oral preparation is prepared, for example, by mixing an active ingredient with an excipient (such as lactose, mannitol, glucose, microcrystalline cellulose and starch), a bonding agent (such as hydroxypropyl cellulose, polyvinylpyrrolidone and magnesium aluminometasilicate), a disintegrating agent (such as calcium cellulose glycolate), a lubricant (such as magnesium stearate), a stabilizer, a solubilizing agent (such as glutamic acid and aspartic acid) and the like by a routine procedure. In addition, if necessary, the active ingredient may be coated with a coating agent (such as white soft sugar, gelatin, hydroxypropyl cellulose and hydroxypropyl methylcellulose phthalate) or may be coated with two or more layers.

The external preparation as a parenteral preparation is prepared by a known method or according to a normally used formulation. For example, an ointment is prepared by triturating or melting an active ingredient in a base. An ointment base is selected from those which are known and those which are normally used. For example, one selected from the followings is used or two or more kinds selected from the followings are used by being mixed together: a higher fatty acid or a higher fatty acid ester (such as adipic acid, myristic acid, palmitic acid, stearic acid, oleic acid, an adipate, a myristate, a palmitate, a stearate and an oleate), waxes (such as beeswax, whale wax and ceresin), a surface-active agent (such as a polyoxyethylene alkyl ether phosphoric ester), a higher alcohol (such as cetanol, stearyl alcohol and cetostearyl alcohol), a silicone oil (such as dimethyl polysiloxane), hydrocarbons (such as hydrophilic petrolatum, white petrolatum, purified lanolin and liquid paraffin), glycols (such as ethylene glycol, diethylene glycol, propylene glycol, polyethylene glycol and macrogol), a vegetable oil (such as castor oil, olive oil, sesame oil and turpentine oil), an animal oil (such as mink oil, egg-yolk oil, squalane and squalene), water, an absorption promoter and an agent for preventing skin rash. In addition, a moisturizing agent, a preservative, a stabilizing agent, an antioxidant, a flavoring agent and the like may be contained.

The injection preparation as a parenteral preparation includes a solution, a suspension, an emulsion and a solid injection preparation which is used at the time of use by being dissolved or suspended in a solvent. The injection preparation is used, for example, by dissolving, suspending or emulsifying an active ingredient in a solvent. Examples of the solvent used include distilled water for injection, saline, a vegetable oil, alcohols such as propylene glycol, polyethylene glycol and ethanol and the like as well as a mixture thereof. In addition, the injection preparation may contain a stabilizer, a solubilizing agent (such as glutamic acid, aspartic acid and polysorbate 80 (registered trademark)), a suspending agent, an emulsifying agent, an analgesic, a buffer agent, a preservative and the like. The above-described injection preparation is prepared by being sterilized at the final process or by an aseptic manipulation method. In addition, the above-described injection preparation can be also used by preparing a sterile solid preparation, for example, a lyophilized preparation, and dissolving the sterile solid preparation in sterilized or sterile distilled water for injection or other solvent before use of the preparation.

In the present invention, Compound A or various acid addition salts of Compound A can be produced according to, for example, examples described later and methods similar thereto. When recrystallizing, seed crystal may or may not be used.

### EXAMPLES

The present invention will be described in detail with reference to examples and biological examples hereinbelow, but the present invention is not limited thereto.

The compound names of the present invention and the compound names shown in the examples were named by ACD/Name (version 6.00, manufactured by Advanced Chemistry Development Inc.) or Chemdraw Ultra (version 12.0, manufactured by Cambridge Soft).

Almost all chemicals used in operations of reactions, extraction, drying, column chromatography and ¹H-NMR spectrum measurement were commercially available unless otherwise specified.

δ Values for chemical shift values in ¹H nuclear magnetic resonance spectrum (¹H-NMR spectrum) was expressed in ppm. Tetramethylsilane (TMS) was used as a reference substance. Abbreviations s, d, t, q, quin., m, br, and dd for signal splitting patterns mean singlet, doublet, triplet, quartet, quintet, multiplet, broad, and double doublet, respectively.

Mass spectra and LCMS analysis were performed using Ultra Performance LC and ACQUITY SQD ESI mass spectrometers manufactured by Waters Corporation.

A position of chromatographic separation indicates an eluting solvent used.

A solvent in parentheses shown in NMR part indicates a solvent used in measurement.

LC-MS/ELSD was carried out in the following conditions: {Column: Waters ACQUITY C18(2) column (particle size: 1.7 × 10⁻⁶ m; column length: 30 × 2.1 mm I.D.); flow rate: 1.0 mL/min; mobile phase (A): 0.1% formic acid aqueous solution; mobile phase (B): 0.1% formic acid-acetonitrile solution; gradient (a ratio of mobile phase (A) : mobile phase (B) is described): [0 min] 95 : 5; [0.1 min] 95 : 5; [1.2 min] 5 : 95; [1.4 min] 5 : 95; [1.41 min] 95 : 5; [1.5 min] 95 : 5; detector: UV (detection wavelength: 254 nm).

### Example 1:

### 1-Benzyl 2-methyl (2S,4R)-4-hydroxy-1,2-pyrrolidindicarboxylate

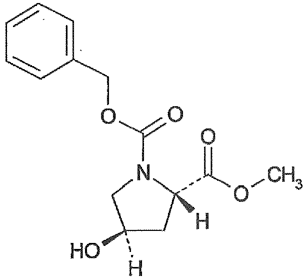

(2S,4R)-Methyl-hydroxypyrrolidine-2-carboxylate hydrochloride (132 g), water (675 mL) and 1,4-dioxane (675 mL) were added and the solution was cooled to 0°C, then, sodium hydrogen carbonate (81.3 g) was added thereto over 30 minutes. Thereafter, benzyl chloroformate (122 mL) was added to the reaction solution over 45 minutes. After stirring the reaction solution at 0°C for 2 hours, the reaction solution was returned to room temperature and further stirred at room temperature for 17 hours. Dioxane in the reaction solution was removed under vacuum, and the remaining aqueous solution was extracted with dichloromethane. The mixed organic layers were washed with 1 N hydrochloric acid and saturated saline. The obtained solution was dried over sodium sulfate, and filtered, and then the filtrate was concentrated. The obtained residue was purified by column chromatography (dichloromethane : methanol = 98 : 2) to give the title compound (158 g, 78%) having the following physical properties.
Properties: Colorless oily substance;
¹H NMR (CD₃OD, 500 MHz): δ 7.36-7.30 (m, 5H), 5.17 (d, 0.5H), 5.12 (s, 1H), 5.00 (d, 0.5H), 4.50-4.42 (m, 1H), 4.43-4.48 (m, 1H), 3.60-3.30 (m, 2H), 3.56 (s, 3H), 2.32-2.22 (m, 1H), 2.12-1.98 (m, 1H).

### Example 2:

### (2S,4R)-1-Benzyl-2-methyl-4-(trifluoromethylsulfonyloxy)pyrrolidine-1,2-dicarboxylate

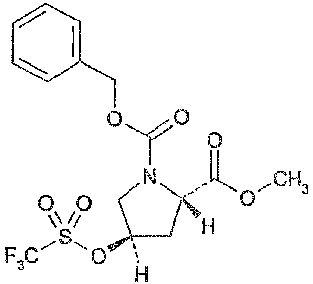

The compound (138 g) produced in Example 1, pyridine (80 mL) and anhydrous dichloromethane (1.30 L) were added. After cooling the solution to -78°C, trifluoromethanesulfonic anhydride (125 mL) was added dropwise thereto over 45 minutes so that the temperature of the solution became -65°C or less. The reaction solution was warmed to -5°C over 1 hour and stirred at that temperature for 2 hours. After concentrating the reaction solution to half the volume under vacuum, the reaction solution was extracted with methyl tert-butyl ether and diluted with dichloromethane. The combined organic layers were washed with 10% aqueous copper sulfate/pentahydrate solution, saturated ammonium chloride aqueous solution and saturated saline. The solution was dried over sodium sulfate and filtered, and then the filtrate was concentrated to give the title compound (189 g, 93%) having the following physical properties.
Properties: Yellow oily substance;
¹H NMR (CD₃OD, 500 MHz; rotational isomers exist): δ 7.40-7.25 (m, 5H), 5.69 (s, 1H), 5.22-4.95 (m, 2H), 4.58-4.50 (m, 1H), 4.08-3.42 (m, 4H), 2.84-2.68 (m, 1H), 2.50-2.36 (m, 1H), 2.33-2.24 (m, 1H).

### Example 2 (1):

### 1-Benzyl 2-methyl (2S,4R)-4-{[(4-nitrophenyl)sulfonyl]oxy}pyrrolidine-1,2-dicarboxylate

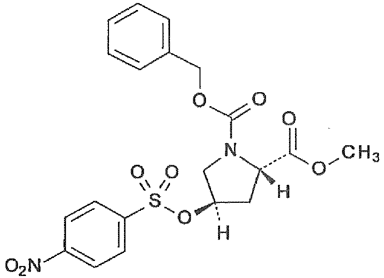

The compound (288 g) produced in Example 1, triethylamine (287 mL) and ethyl acetate (720 mL) were added. An ethyl acetate solution (720 mL) of 4-nitrobenzenesulfonyl chloride (274 g) was added dropwise thereto at an internal temperature of 20 to 40°C. Thereafter, the reaction solution was stirred overnight. After adding ethyl acetate (860 mL) to the reaction solution, the reaction was stopped with a 10% aqueous sodium carbonate solution (1.44 L). After separation, the organic layer was washed with 10% aqueous sodium carbonate solution and 20% saline solution. After adding n-heptane (580 mL) to the organic layer and stirring the mixture for 1 hour, n-heptane (2.88 L) was further added. After stirring the mixture for 1 hour, the precipitate was filtered. The wet crystals were vacuum dried at 60°C for 12 hours to give the title compound (376 g, 82%) having the following physical properties.
Properties: White solid;
¹H NMR (CDCl₃, 400 MHz; rotational isomers exist): δ 8.41-8.35 (m, 2H), 8.10-8.06 (m, 2H), 7.39-7.27 (m, 5H), 5.22-4.99 (m, 3H), 4.52-4.44 (m, 1H), 3.82-3.67 (m, 3.5H), 3.55 (s, 1.511), 2.65-2.51 (m, 1H), 2.29-2.20 (m, 1H).
MASS(ESI, Pos.): 465 (M+H)⁺.

### Example 3:

### 1-Benzyl 2-methyl (2S,4S)-4-[4-(methylsulfonyl)-1-piperazinyl]-1,2-pyrrolidindicarboxylate

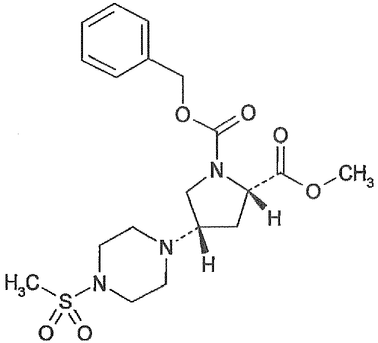

The compound (189 g) produced in Example 2, N,N-diisopropylethylamine (160 mL) and dried tert-butanol (1.45 L) were added, 1-methanesulfonyl piperazine (226 g) was added thereto under an argon atmosphere at room temperature, and the mixture was stirred. Thereafter, the reaction solution was refluxed for 16 hours. After cooling, the obtained reaction solution was concentrated to an orange oily substance. The oily substance was diluted with dichloromethane (2.0 L) and washed with water. The aqueous layer was extracted with ethyl acetate, and the combined organic layers were dried over sodium sulfate and filtered. The filtrate was concentrated and the obtained residue was purified by column chromatography (dichloromethane : methanol = 98 : 2) to give the title compound (145 g, 74%) having the following physical properties.

The title compound can also be obtained by using the compound produced in Example 2 (1). When the compound (150 g) produced in Example 2 (1) was used, sodium hydrogen carbonate (41 g), acetonitrile (300 mL), and 1-methanesulfonyl piperazine (80 g) are added, and the mixture is refluxed overnight, whereby the title compound can be obtained.
Properties: Off-white solid;
¹H NMR (CDCl₃, 500 MHz; containing approximately 20% diastereomer (2R,4S), and rotational isomers exist): δ 7.37-7.28 (m, 5H), 5.18 (d, 1H), 5.11-5.07 (m, 0.5H), 5.02-4.98 (m, 0.5H), 4.41-4.30 (m, 1H), 3.98-3.94 (m, 0.5H), 3.88-3.84 (m, 0.5H), 3.75 (s, 1.5H), 3.55 (s, 1.5H), 3.32-3.28 (m, 1H), 3.27-3.20 (m, 4H), 2.90-2.80 (m, 1H), 2.77 (s, 3H), 2.65-2.45 (m, 5H), 1.92-1.81 (m, 1H);
MASS(ESI, Pos.): 426 (M+H)⁺.

### Example 4:

### (2S,4S)-1-[(Benzyloxy)carbonyl]-4-[4-(methylsulfonyl)-1-piperazinyl]-2-pyrrolidinecarboxylic acid

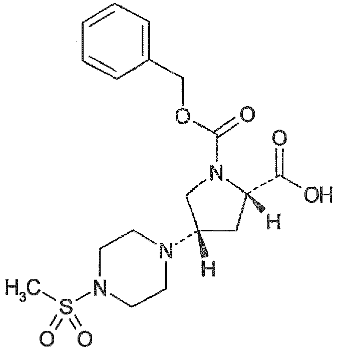

The compound (145 g) produced in Example 3 and tetrahydrofuran (790 mL) were added, and the solution was cooled to -3°C, then an aqueous solution (790 mL) of lithium hydroxide (32.6 g) was added dropwise thereto over 1 hour or more so that the temperature of the solution became 0°C or less. The reaction solution was returned to room temperature and further stirred at room temperature for 15 hours. Tetrahydrofuran in the reaction solution was removed under vacuum, and water (1 L) was added thereto. The remaining solution was washed with methyl tert-butyl ether and 2 N hydrochloric acid (275 mL) was added thereto to bring the pH to 3 to 4. Dichloromethane was added to the aqueous layer for extraction. In addition, the precipitate formed during the extraction was collected by filtration. After removing half the amount of the dichloromethane, more precipitates were generated, so the precipitates were collected by filtration. The precipitates collected by filtration were combined and vacuum dried at room temperature for 17 hours to give the title compound (82.1 g, 59%) having the following physical properties.
Properties: White solid (containing no diastereomer (2R,4S));
¹H NMR (CDCl₃, 400 MHz; rotational isomers exist): δ 7.36-7.33 (m, 5H), 5.18-5.12 (m, 2H), 4.43-4.36 (m, 1H), 3.98-3.82 (m, 2H), 3.37-3.17 (m, 4H), 3.00-2.91 (m, 1H), 2.82-2.71 (m, 6H), 2.64-2.49 (m, 2H), 2.24-2.12 (m, 1H), 1.97-1.85 (m, 1H);
MASS(ESI, Pos.): 412 (M+H)⁺.

### Example 5:

### Benzyl (2S,4S)-2-[(4-{[(2-methyl-2-propanoyl)oxy]carbonyl}phenyl)carbamoyl]-4-[4-(methylsulfonyl)-1-piperazinyl]-1-pyrrolidinecarboxylate

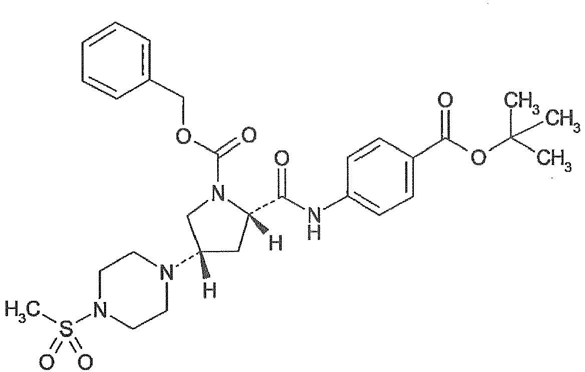

The compound (82.1 g) produced in Example 4, tert-butyl-4-aminobenzoate (77.2 g) and anhydrous pyridine (1.50 L) were added. After cooling the solution to -4°C under a nitrogen gas atmosphere, N-(3-dimethylaminopropyl)-N'-ethylcarbodiimide hydrochloride (306 g) was added over 15 minutes. The reaction solution was returned to room temperature and further stirred at room temperature for 15 hours. The reaction solution was removed under vacuum, and dichloromethane (2 L) was added thereto. The reaction solution was washed with 10% aqueous copper sulfate/pentahydrate solution. The filtrate was washed with water, saturated ammonium chloride aqueous solution and saturated saline. The solution was dried over sodium sulfate and filtered. The filtrate was concentrated to a dark yellow oily substance. The obtained residue was purified by column chromatography (dichloromethane : methanol = 100 : 0 → 97.5 : 2.5) to give the title compound (108 g) having the following physical properties.
Properties: Yellow solid;
¹H NMR (CDCl₃, 500 MHz): δ 7.90 (d, 2H), 7.56-7.10 (m, 3H), 7.26-7.21 (m, 3H), 7.16-7.13 (m, 2H), 5.29-5.01 (m, 2H), 4.52-4.40 (m, 1H), 3.87-3.78 (m, 1H), 3.18-3.01 (m, 4H), 2.85 (quin, 1H), 2.82-2.50 (m, 7H), 2.35 (s, 3H), 1.66 (s, 9H);
MASS(ESI, Pos.): 587 (M+H)⁺.

### Example 6:

### 2-Methyl-2-propanoyl 4-[({(2S,4S)-4-[4-(methylsulfonyl)-1-piperazinyl]-2-pyrrolidinyl}carbonyl)amino]benzoate

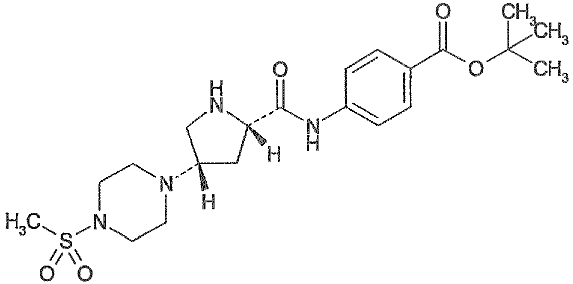

Under a nitrogen gas atmosphere, 10% palladium carbon (ca. 50% wet; 4.82 g) was added to an ethanol solution (200 mL) of the compound (21.7 g) produced in Example 5, and then the mixture was stirred under hydrogen pressure (40 psi) for 1.5 hours. The reaction solution was diluted with dichloromethane (300 mL) and filtered, and the filtrate was concentrated to give the title compound (16.6 g, 99%) having the following physical properties.
Properties: Gray solid;
¹H NMR (CDCl₃, 500 MHz): δ 9.59 (s, 1H), 7.94 (d, 2H), 7.63 (d, 2H), 3.94 (t,Hz, 1H), 3.28-3.23 (m, 1H), 3.16-3.11 (m, 4H), 2.90-2.85 (m, 1H), 2.83-2.77 (m, 1H), 2.63 (s, 3H), 2.62-2.57 (m, 2H), 2.54-2.50 (m, 2H), 2.51-2.47 (m, 1H), 2.03-1.97 (m, 1H), 1.58 (s, 9H);
MASS(ESI, Pos.): 453 (M+H)⁺.

### Example 7:

### 2-Methyl-2-propanoyl 4-[({(2S,4S)-1-[4-(N-{[(2-methyl-2-propanoyl)oxy]carbonyl}carbamimidoyl)benzoyl]-4-[4-(methylsulfonyl)-1-piperazinyl]-2-pyrrolidinyl}carbonyl)amino]benzoate

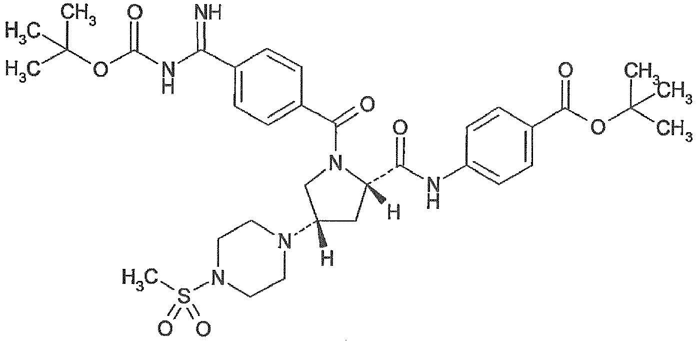

The compound (10.0 g) produced in Example 6, N,N-diisopropylethylamine (11.5 mL) and N-tert-butoxycarbonyl-4-amidinobenzoic acid (6.42 g; manufactured according to the production method described in Reference Example 12 d of US Pat. No. 5,973,188) were added to a mixed solution of dry acetonitrile (250 mL) and dichloromethane (100 mL), and the mixture was cooled to -45°C under a nitrogen gas atmosphere, and HATU (O-(7-azabenzotriazol-1-yl)-1,1,3,3-tetramethyluronium hexafluorophosphate; 10.1 g) was added over 10 minutes. The reaction solution was returned to room temperature and further stirred at room temperature for 1 hour. The reaction solution was removed under vacuum, and the obtained residue was purified by column chromatography (dichloromethane : isopropanol = 100 : 0 → 90 : 10) to give the title compound (9.00 g, 58%) having the following physical properties.
Properties: Off-white solid;
¹H NMR (CDCl₃, 500 MHz): δ 9.45 (s, 1H), 7.95-7.85 (m, 4H), 7.62-7.54 (m, 4H), 5.00-4.90 (m, 1H), 3.71-3.62 (m, 1H), 3.44-3.37 (m, 1H), 3.28-3.16 (m, 5H), 2.89-2.80 (m, 1H), 2.77 (s, 3H), 2.70-2.54 (m, 4H), 2.50-2.32 (m, 3H), 1.58 (s, 9H), 1.54 (s, 9H);
MASS(ESI, Pos.): 699 (M+H)⁺.

### Example 8:

### 4-({(4S)-1-(4-Carbamimidoylbenzoyl)-4-[4-(methylsulfonyl)piperazin-1-yl]-L-prolyl} amino) benzoic acid dihydrochloride

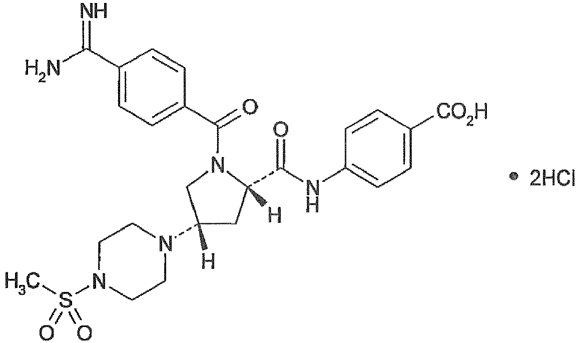

The compound (36.2 g) produced in Example 7 was dissolved in anhydrous dichloromethane (510 mL), and trifluoroacetic acid (118.1 g) was added dropwise thereto at 0°C. The reaction solution was stirred at room temperature for 22 hours and then concentrated under reduced pressure. The residue was azeotroped with dichloromethane and acetonitrile until it became a viscous orange oily substance. The oily substance was dissolved in acetonitrile (650 mL), and hydrogen chloride gas was bubbled for 5 minutes. After stirring the solution for 30 minutes, a white precipitate was formed. The reaction solution was concentrated to about half the volume. Acetonitrile (330 mL) was added to the reaction solution, and the reaction solution was concentrated again to about half the volume. The white precipitate was filtered off and washed with acetonitrile. The remaining solution was lyophilized for 4 days to give the title compound (23.9 g, 79%) having the following physical properties.
Properties: Amorphous white solid;
¹H NMR (D₂O, 500 MHz, rotational isomers exist): δ 7.99 (1H), 7.86 (t, 2H), 7.78 (d, 1H), 7.72 (d, 1H), 7.63-7.60 (m, 2H), 7.17 (dd, 1H), 4.83 (dd, 0.5H), 4.77 (dd, 0.5H), 4.45 (dd, 0.5H), 4.17-4.08 (m, 1H), 3.98-3.91 (m, 1H), 3.85 (dd, 0.5H), 3.59-3.42 (m, 7H), 3.36-3.32 (m, 1H), 3.07-2.97 (m, 4H), 2.37-2.30 (m, 1H);
MASS(ESI, Pos.): 543 (M+H)⁺.
Purity (HPLC): 99% or more (retention time: 6.5 minutes).

### Example 9:

### 4-({(4S)-1-(4-Carbamimidoylbenzoyl)-4-[4-(methylsulfonyl)piperazin-1-yl]-L-prolyl}amino) benzoic acid pentahydrate

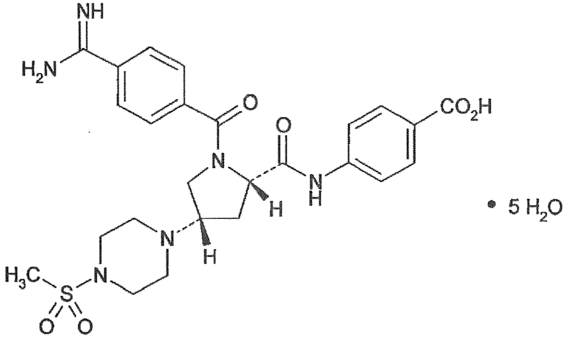

The compound (37.0 g) produced in Example 8 was dissolved in water (740 mL), 28% aqueous ammonium solution (10.6 mL) was added thereto at 25°C, and the mixture was stirred for 1 minute. After white crystals were precipitated, the reaction solution was stirred at 4°C for 30 minutes. The precipitated crystals were filtered off and washed with water (74 mL). Water (185 mL) was added to the filtered crystals, and the suspension solution was stirred at 4°C for 40 minutes. The suspension solution was filtered off, and precipitated crystals were washed with water (74 mL). The crystals were dried under reduced pressure at room temperature for 14 hours and then left to stand in an environment of 74% humidity for 7 hours to give the title compound (32.2 g, 85%) having the following physical properties.
Shape: Crystalline white solid;
¹H NMR (DMSO-d₆, 300 MHz, rotational isomers exist): δ 10.22 (br. s, 1H), 9.54-8.30 (br, 2H), 7.98-7.46 (m, 8H), 6.98-6.81 (m, 1H), 4.75-4.54 (m, 1H), 3.73-3.48 (m, 2H), 3.41-2.29 (m, 13H), 1.88-1.60 (m, 1H).

### Example 10:

### 4-(1(4S)-1-(4-Carbamimidoylbenzoyl)-4-[4-(methylsulfonyl)piperazin-1-yl]-L-prolyl}amino) benzoic acid monoacetate

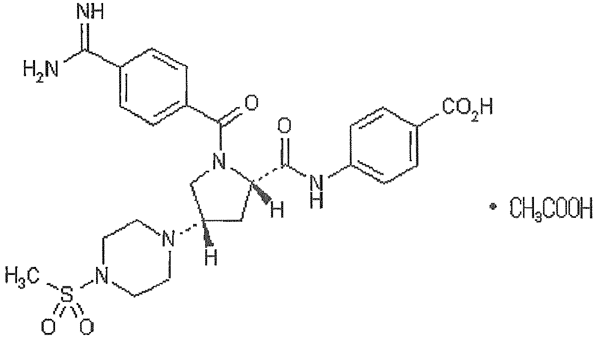

The compound (0.12 g) produced in Example 9 was suspended in 1% acetic acid-ethyl acetate solution (3.75 mL), acetic acid (0.25 mL) was gradually added thereto at 25°C, and the mixture was stirred for 30 minutes. After further addition of acetic acid (2.3 mL), suspended crystals were filtered off. The crystals were dried under reduced pressure at 60°C for 16 hours to obtain the title compound (0.11 g, 100%) having the following physical properties.
Shape: Crystalline white solid;
¹H NMR (D₂O, 300 MHz, rotational isomers exist): δ 7.93 (dd, 2H), 7.83-7.75 (m, 3H), 7.66 (d, 1H), 7.59 (d, 1H), 7.12 (d, 1H), 4.32 (dd, 0.5H), 4.13 (dd, 0.5H), 3.90 (m, 1H), 3.68 (dd, 0.5H), 3.58 (dd, 1H) 3.48-3.16 (m, 5H), 3.04 (s, 1.5H), 3.00 (s, 1.5H), 2.98-2.63 (m, 5H), 2.98-2.63 (m, 5H), 2.15-2.05 (m, 1.5H), 2.03 (s, 3H);
MASS(FAB, Pos.): 543 (M + H -AcOH)⁺.

### [Study of crystallization of Compound A]

### (1) Study of crystallization with automated crystallization device:

Using the compound produced in Example 9, possibility of crystallization of salts of Compound A was studied.

Using 12 types of acid counters (hydrochloric acid (1 equivalent), sulfuric acid (1 equivalent), citric acid (1 equivalent), tartaric acid (1 equivalent), phosphoric acid (1 equivalent), fumaric acid (1 equivalent), methanesulfonic acid (1 equivalent), 4-toluenesulfonic acid (hereinafter sometimes abbreviated as p-toluenesulfonic acid or tosyl acid; 2 equivalents), benzenesulfonic acid (besyl acid; 1 equivalent), besyl acid (2 equivalents), ethanedisulfonic acid (1/2 equivalents), isethionic acid (1 equivalent)), and using 8 types of solvents (acetone, acetonitrile, ethyl acetate, methanol, 70% ethanol aqueous solution, toluene, heptane, methyl tert-butyl ether (MTBE)), crystallization of salts of Compound A was studied, using an automated crystallization device (Core Module (X) manufactured by Freeslate). Setting four conditions of slurry method (natural cooling from 55°C to room temperature), cooling method (55°C to 10°C, -10°C/h), precipitation method (55°C dissolution, precipitation at room temperature), and evaporation concentration method (55°C dissolution, evaporation at room temperature) as crystallization methods, 288 crystallization conditions were set for each salt by combining the solvent and the crystallization method.

As a result, a slight crystalline reflection peak was observed from salts of tosyl acid (2 equivalents) and besyl acid (2 equivalents). However, when attempts were made to reacquire crystals under the conditions in which crystals were obtained for both of these salts, crystals could not be obtained due to lack of reproducibility. Therefore, a more comprehensive manual study of crystallization was carried out.

### (2) Manual study of crystallization:

Using 12 types of acid counters (hydrochloric acid (1 equivalent), sulfuric acid (0.5 equivalents, 1 equivalent, 2 equivalents), phosphoric acid (1 equivalent, 2 equivalents), mesyl acid (1 equivalent, 2 equivalents), ethanesulfonic acid (1 equivalent, 2 equivalents), besyl acid (1 equivalent, 2 equivalents), tosyl acid (1 equivalent, 2 equivalents), isethionic acid (1 equivalent, 2 equivalents), ethanedisulfonic acid (0.5 equivalents, 1 equivalent), (+)-10-camphorsulfonic acid (1 equivalent), (-)-10-camphorsulfonic acid (1 equivalent), hydrobromic acid (1 equivalent)), and using 17 types of solvents (water, methanol, ethanol, 1-propanol, 2-propanol, methanol: water = 9 : 1, ethanol: water = 9 : 1, 1-propanol: water = 9 : 1, 2-propanol : water = 9 : 1, acetonitrile, acetone, acetonitrile : water = 9 : 1, acetone : water = 9: 1, tetrahydrofuran, ethyl acetate, MTBE, toluene), a manual study of crystallization was carried out according to the following procedures.
1) Weigh 10 mg (5 mg if necessary) of the compound produced in Example 9 into a disposable culture tube (manufactured by AGC Inc., 10 × 75 mm, catalog number: 9830-1007).
2) Set outside temperature to 80°C (50°C only when acetone is used as the solvent), and add the solvent until the compound is dissolved. When the compound is not dissolved, add the solvent up to 80 v/w (volume/weight).
3) Allow the solutions to stand overnight at room temperature.
4) For those that did not precipitate, allow them to stand in a refrigerator (7°C).
5) For those that did not precipitate, remove a lid and let the solvent evaporate naturally.

As a result, a crystalline reflection peak was observed only when tosyl acid (2 equivalents) and besyl acid (2 equivalents) were used as the acid counters and tetrahydrofuran was used as the solvent.

Regarding the study results of other acid counters, it was consistent with the powder X-ray diffraction spectrum of the compound (pentahydrate) produced in Example 9 described above without forming a salt, or it formed a salt but only showed an amorphous form.

Next, crystallization was studied in a mixed solvent containing alcohol, using tosyl acid (2 equivalents) and besyl acid (2 equivalents) as the acidic counters.

Table 1 below shows study results when using tosyl acid (2 equivalents) as the acid counter according to the above procedures.

**[Table 1]**

| Solvent (Mixing ratio (w/w)) | Solvent amount (µL) | Shape |
|---|---|---|
| Acetonitrile/methanol (7/1) | 320 | Amorphous |
| Acetone/methanol (15/2) | 340 | Amorphous |
| Tetrahydrofuran/methanol (15/2) | 340 | Crystal form |
| Ethyl acetate/methanol (3/1) | 160 | Amorphous |
| MTBE/methanol (3/2) | 100 | Amorphous |
| Toluene/methanol (4/1) | 200 | Amorphous |
| Acetonitrile/ethanol (7/1) | 640 | Amorphous |
| Acetone/ethanol (13/2) | 600 | Amorphous |
| Tetrahydrofuran/ethanol (58/8) | 660 | Crystal form |
| Ethyl acetate/ethanol (5/4) | 180 | Amorphous |
| MTBE/ethanol (3/4) | 140 | Amorphous |
| Toluene/ethanol (7/4) | 220 | Amorphous |

As a result of the studies, it was found that ditosylate and dibesylate of Compound A show a crystal form only when a solvent containing tetrahydrofuran is used. Detailed crystallization processes will be described in Examples 11 and 12.

### Example 11:

### 4-({(4S)-1-(4-Carbamimidoylbenzoyl)-4-[4-(methylsulfonyl)piperazin-1-yl]-L-prolyl}amino) benzoic acid di(4-toluenesulfonate)

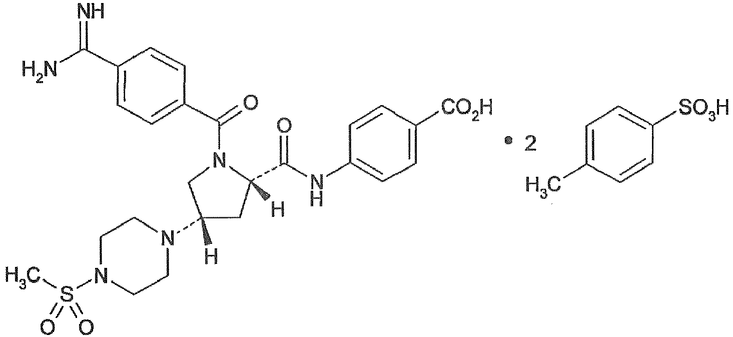

The compound (31.5 g) produced in Example 9 and 4-toluenesulfonic acid monohydrate (18.9 g) were dissolved in ethanol (110 mL) and stirred at 50°C for 10 minutes. Ethanol (15.8 mL) was further added thereto, and the mixture was stirred at 50°C for 5 minutes. The solution was filtered, ethanol (15.8 mL) was added to the filtered solution, and the mixture was warmed to 45°C over 5 minutes, then tetrahydrofuran (536 mL) was added thereto over 3 minutes. After stirring at 50°C for 10 minutes, the mixture was stirred at 25°C overnight. The precipitated crystals were filtered off and washed with tetrahydrofuran (158 mL). When the surface of the crystal was exposed, the surface of the crystal was flowed with argon gas, and the obtained crystals were dried in a desiccator for 3 hours at room temperature. Further, the obtained crystals were dried under reduced pressure in a specimen dryer at an internal temperature of 55°C for 48 hours, an internal temperature of 60°C for 12 hours, and an internal temperature of 75°C for 36 hours to give the title compound (41.1 g, 90%) having the following physical properties.
Properties: Crystalline white solid;
¹H NMR (DMSO-d₆, 300 MHz, rotational isomers exist): δ 10.63 (br. s, 1H), 9.41 (br. s, 2H), 9.03 (br. s, 2H), 7.96-7.87 (m, 4H), 7.82-7.70 (m, 4H), 7.62-7.54 (m, 4H), 7.35-7.26 (m, 5H), 4.71 (t, 1H), 4.24-2.62 (m, 15H), 2.21-1.94 (m, 1H);
MASS(ESI, Pos.): 543 (M+H)⁺.

### Example 12:

### 4-({(4S)-1-(4-Carbamimidoylbenzoyl)-4-[4-(methylsulfonyl)piperazin-1-yl]-L-prolyl}amino) benzoic acid dibenzenesulfonate

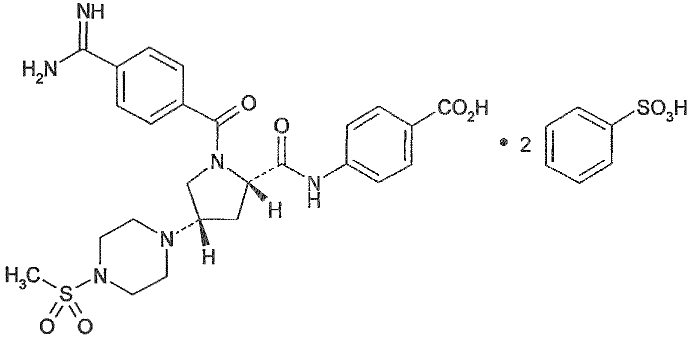

The same procedure as in Example 11 was carried out by using the compound (0.150 g) produced in Example 9, using besyl acid (0.075 g) instead of 4-toluenesulfonic acid monohydrate, and using 2.1 mL and 3.9 mL of ethanol and tetrahydrofuran, respectively, to give the title compound (0.189 g, 93%) having the following physical properties.
Properties: Crystalline white solid
¹H NMR (DMSO-d₆, 300 MHz, rotational isomers exist): δ 10.63 (br. s, 1H), 9.41 (br. s, 2H), 9.03 (br. s, 2H), 7.96-7.87 (m, 4H), 7.82-7.70 (m, 4H), 7.62-7.54 (m, 4H), 7.35-7.26 (m, 5H), 4.71 (t, 1H), 4.24-2.62 (m, 15H), 2.21-1.94 (m, 1H);
MASS(ESI, Pos.): 543 (M+H)⁺.

### [Measurement of physical property data]

Physical property data of ditosylate, dibesylate, monoacetate and pentahydrate of Compound A for which crystals could be acquired were measured under the following conditions.

### (1) Powder X-ray diffraction spectrum:

Ditosylate and dibesylate were measured under Condition 1, and monoacetate and pentahydrate were measured under Condition 2.

### Condition 1

Device: SmartLab manufactured by Rigaku Corporation
Target: Cu
Voltage: 45 kV
Current: 200 mA
Condition 2
Device: D8 DISCOVER with GADDS manufactured by BRUKER axs,
Target: Cu,
Voltage: 40 kV,
Current: 40 mA.

### (2) Differential scanning calorimetry (DSC):

Device: DSC822e Differential Scanning Calorimeter manufactured by METTLER TOLEDO,
Sample amount: 5.75 mg (ditosylate), 0.86 mg (dibesylate), 2.41 mg (monoacetate), 2.91 mg (pentahydrate)
Sample cell: Aluminum pan 40 µL,
Nitrogen gas flow rate: 40 ml/min,
Heating rate: 10°C/min (25 to 280°C).

In the present invention, each crystal form of Compound A or various acid addition salts of Compound A is specified by physicochemical data described in the present specification, but each spectral data can slightly vary due to its nature, so it should not be understood exactly. For example, in powder X-ray diffraction spectrum data, due to its nature, diffraction angle (20) and overall pattern are important in determination of crystal identity, and relative intensity can slightly vary depending on the direction of crystal growth, particle size, and measurement conditions. Also in DSC data, overall pattern is important in the determination of crystal identity, and it can slightly vary depending on the measurement conditions. Therefore, in the compound of the present invention, a compound having an overall similar pattern to the powder X-ray diffraction spectrum or DSC is included in the compound of the present invention.

In the present specification, the description of the diffraction angle (20 (degrees)) in the powder X-ray diffraction pattern and the temperature (°C) in the DSC analysis means that an error range usually allowed in the data measurement method is included, and means to be approximately the diffraction angle and the temperature. For example, the "about" or "substantially the same" diffraction angle (20 (degrees)) in powder X-ray diffraction is ± 0.2 degrees in one embodiment and ± 0.1 degrees in yet another embodiment. The "about" of onset temperature (°C) and endothermic peak temperature (°C) in DSC analysis is ± 5°C in one embodiment, ± 2°C in another embodiment, and ± 1°C in yet another embodiment.

The crystals of ditosylate of Compound A showed, in a powder X-ray diffraction spectrum, a powder X-ray diffraction spectrum shown in Fig. 1 and diffraction angles (2θ) and relative intensities shown in Table 2. In addition, the crystals of ditosylate of Compound A showed, in DSC, an endothermic start at about 216°C and an endothermic peak at about 228°C as shown in a chart shown in Fig. 5. The endothermic peak at about 228°C corresponds to melting of the crystal.

**[Table 2]**

| Diffraction angle (2θ) | Relative intensity (%) |
|---|---|
| 6.19 | 100 |
| 7.75 | 11 |
| 9.41 | 64 |
| 11.74 | 11.5 |
| 15.55 | 26.6 |
| 16.01 | 29.1 |
| 16.26 | 12 |
| 17.14 | 43.7 |
| 18.55 | 48.8 |
| 20.03 | 17.5 |
| 20.26 | 16.3 |
| 21.28 | 26 |
| 22.37 | 22.4 |
| 23.03 | 24.6 |
| 23.53 | 10.6 |
| 23.82 | 11.6 |
| 24.14 | 11.8 |
| 24.69 | 11 |
| 25.91 | 13 |
| 27.19 | 15.7 |

The crystals of dibesylate of Compound A showed, in a powder X-ray diffraction spectrum, a powder X-ray diffraction spectrum shown in Fig. 2 and diffraction angles (2θ) and relative intensities shown in Table 3. In addition, the crystals of dibesylate of Compound A showed, in DSC, an endothermic start at about 203°C and an endothermic peak at about 213°C as shown in a chart shown in Fig. 6. The endothermic peak at about 213°C corresponds to melting of the crystal.

**[Table 3]**

| Diffraction angle (2θ) | Relative intensity (%) |
|---|---|
| 6.24 | 100 |
| 7.73 | 11.3 |
| 11.99 | 14.9 |
| 12.51 | 12.4 |
| 16.21 | 50.3 |
| 17.21 | 33.5 |
| 17.69 | 28.3 |
| 18.6 | 21.2 |
| 18.76 | 23 |
| 19.03 | 14.2 |
| 20.3 | 12.2 |
| 20.78 | 10.9 |
| 21.51 | 18.4 |
| 22.48 | 12 |
| 22.85 | 12.4 |
| 23.14 | 15.6 |

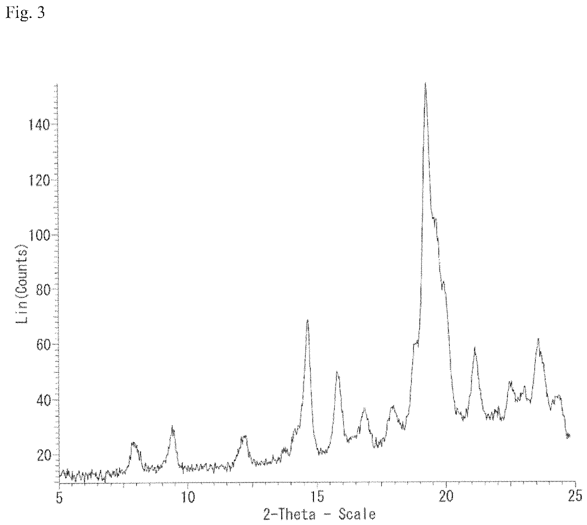

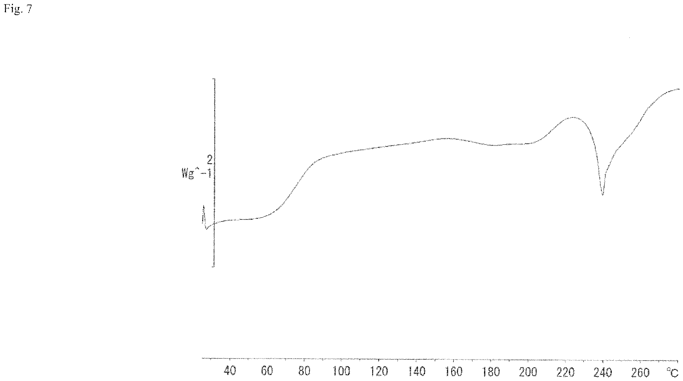

The crystals of monoacetate of Compound A showed, in a powder X-ray diffraction spectrum, a powder X-ray diffraction spectrum shown in Fig. 3 and diffraction angles (2θ) and relative intensities shown in Table 4. Also, the crystals of monoacetate of Compound A showed a chart shown in Fig. 7 in DSC.

**[Table 4]**

| Diffraction angle (20) | Relative intensity (%) |
|---|---|
| 7.88 | 15.9 |
| 9.4 | 19.7 |
| 12.24 | 17.3 |
| 14.66 | 44.4 |
| 15.78 | 32.2 |
| 16.84 | 23.6 |
| 17.96 | 24.2 |
| 19.28 | 100 |
| 21.14 | 37.9 |
| 22.54 | 29.9 |
| 23.04 | 28.6 |
| 23.6 | 39.8 |
| 24.4 | 26.5 |

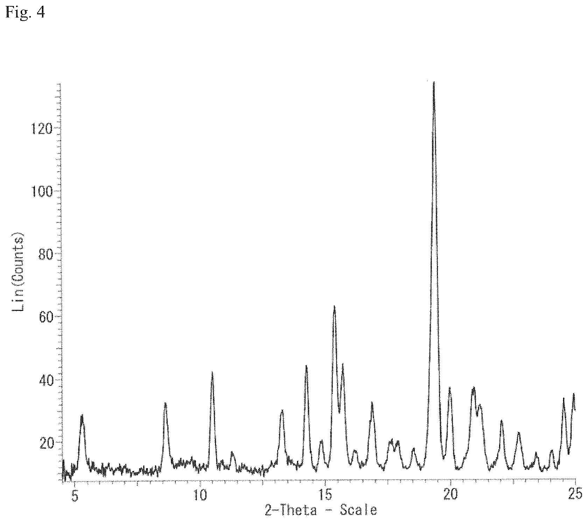

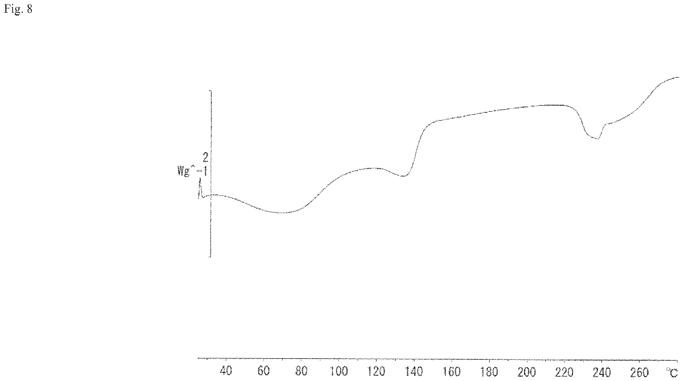

The crystals of pentahydrate of Compound A showed, in a powder X-ray diffraction spectrum, a powder X-ray diffraction spectrum shown in Fig. 4 and diffraction angles (2θ) and relative intensities shown in Table 5. Also, the crystals of pentahydrate of Compound A showed a chart shown in Fig. 8 in DSC.

**[Table 5]**

| Diffraction angle (2θ) | Relative intensity (%) |
|---|---|
| 5.311 | 21.5 |
| 8.635 | 24.2 |
| 10.515 | 31.4 |
| 11.315 | 12.1 |
| 13.282 | 22.2 |
| 14.271 | 32.9 |
| 14.852 | 15.1 |
| 15.397 | 46.8 |
| 15.708 | 33.2 |
| 16.187 | 12.7 |
| 16.893 | 24.1 |
| 17.632 | 15 |
| 17.896 | 14.8 |
| 18.538 | 13 |
| 19.431 | 100 |
| 19.995 | 27.5 |
| 20.929 | 27.5 |
| 21.19 | 23.4 |
| 22.059 | 19.6 |
| 22.737 | 19.7 |
| 23.435 | 12 |
| 24.06 | 12.5 |
| 24.556 | 24.8 |
| 24.944 | 26.1 |

In one embodiment of the invention, each crystal form of Compound A, or the various acid addition salts of Compound A, is substantially pure. The reference to "is substantially pure" means that a particular crystal form accounts for at least 50% of the compounds present. Further, in another embodiment, each crystal form accounts for at least 75%, at least 85%, at least 90%, at least 95%, or about 94% to 98% of Compound A present.

The various acid addition salts of Compound A may exist in an unsolvated form or may exist in a solvated form with a pharmaceutically acceptable solvent such as water or ethanol.

The various acid addition salts of Compound A can also be converted into solvates by a known method. The solvate is preferably a low-toxicity and water-soluble solvate. Examples of the appropriate solvate include a solvate with water (hydrate) and a solvate of an alcohol based solvent (for example, ethanol or the like).

### Physicochemical Example 1: Hygroscopicity evaluation

The isotherm adsorption curve was measured under the following conditions. Ditosylate, monoacetate and pentahydrate of Compound A were measured under Condition 1, and besylate of Compound B was measured under Condition 2.

### (Condition 1)

Device: VT1 SGA-100
Measurement temperature: 25°C
Measurement range: Relative humidity of 5 to 95%
Measurement interval: Relative humidity of 5%

### (Condition 2)

Device: DVS Intrinsic
Measurement temperature: 25°C
Measurement range: Relative humidity of 0 to 95%
Measurement interval: Relative humidity of 5%

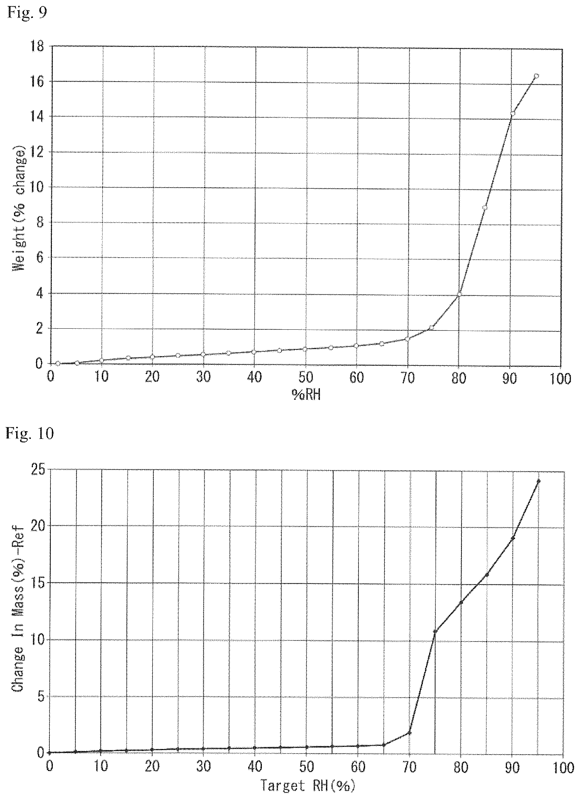

The ditosylate of Compound A showed almost no hygroscopicity at a relative humidity in the range of 5% to 70%, with a weight gain of 1.5 wt% (see Fig. 9).

Further, the dibesylate of Compound A also showed almost no hygroscopicity at a relative humidity in the range of 5% to 70%, with a weight gain of 1.9 wt% (see Fig. 10).

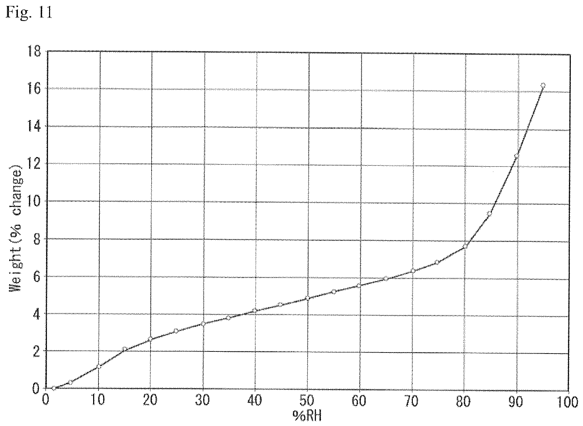

The monoacetate of Compound A showed hygroscopicity at a relative humidity in the range of 5% to 70%, with a weight gain of 6.4 wt% (see Fig. 11).

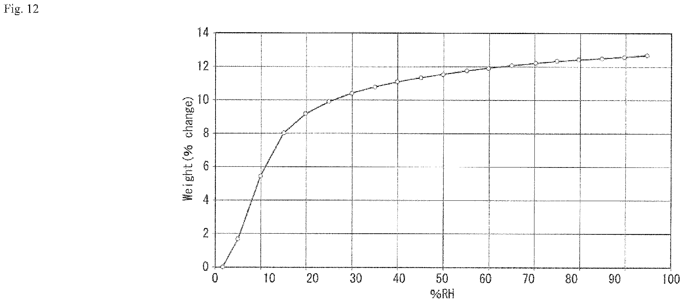

The pentahydrate of Compound A showed hygroscopicity at a relative humidity in the range of 5% to 70%, with a weight gain of 12.2 wt% (see Fig. 12).

From the above results, it was confirmed that the ditosylate and dibesylate of Compound A had extremely low hygroscopicity as compared with the monoacetate and pentahydrate of Compound A.

### Physicochemical Example 2: Chemical stability test

### (1) Residual rate measurement

The chemical stability of crystals of ditosylate, dibesylate, pentahydrate and monoacetate of Compound A was measured by the following method and conditions.

### <Method>

About 1.5 to 3 mg of a test compound was weighed in a lab tube and stored under the following conditions.

After storage, the residual rate (%) of the stored sample under each condition was calculated by HPLC based on the area percentage of main medicine of the sample stored at - 20°C. In addition, appearance was visually observed and compared with the sample stored at -20°C.

### <Preservation conditions and sampling time>

60°C: 1 month
40°C: 2 months
40°C-75% RH (opened): 2 months
2500 Lux:10D, 20D

Comparison object of each sample was stored at -20°C.

### <HPLC Analysis>

### • Sample preparation

An evaluation sample (0.8 mg) was dissolved in a mixed solution of acetonitrile/100 mM NaClO₄ (1/3) about 0.8 mL (sample concentration: 1.0 mg/mL) acetonitrile/100 mmol/L NaClO₄ aq. (pH3.0) (3/1) to prepare a solution of 1.0 mg/mL (concentration as salt).

### <Analysis conditions>

Detector: Ultraviolet absorptiometer (measurement wavelength: 265 nm)
Column: SPELCO Ascentis Express (150 mm × 4.6 mm, S-2.7 µm)
Column temperature: 25°C
Mobile phase: Solution A: 100 mmol/L NaClO₄ aq. (pH 3.0), Solution B: acetonitrile
Gradient condition: A/B = 95/5 (0-10 min) → 80/20 (10-40 min) → 20/80 (40-60 min) → 20/80 (60-65 min)
Flow rate: 1 mL/min
Area measurement range: 34.6 minutes
Injection amount: 5 µL

### <Results>

Table 5 below shows residual rates of crystals of ditosylate, dibesylate, pentahydrate and monoacetate of Compound A.

**[Table 6]**

| | Residual rate (%) | | | |
|---|---|---|---|---|
| | Pentahydrate | Monoacetate | Ditosylate | Dibesylate |
| 60°C-1 month | 23.1 | 97.5 | 100 | 100 |
| 40°C-2 months | 36.3 | 99 | 100 | - |
| 40°C-75%RH-2 months | 37 | 95 | 99.3 | 99.5 |

From the above results, the crystals of pentahydrate and monoacetate of Compound A have a reduced residual rate under the conditions of 40°C-75% and lack chemical stability. On the other hand, the crystals of ditosylate and dibesylate of Compound A showed a residual rate of 99% or more under both conditions, and were extremely excellent in chemical stability.

### (2) Powder X-ray measurement

The physical stability of monoacetate and ditosylate of Compound A was measured by the following method and conditions.

### <Method>

Test compounds were respectively weighed in a lab tube at about 20 mg for monoacetate and about 40 mg for ditosylate, and stored under the following conditions.

### <Preservation conditions>

25°C-60% RH: 2 months

### <Measurement conditions>

Device: D8 DISCOVER with GADDS manufactured by BRUKER axs
Target: Cu
Voltage: 40 kV
Current: 40 mA

### <Results>

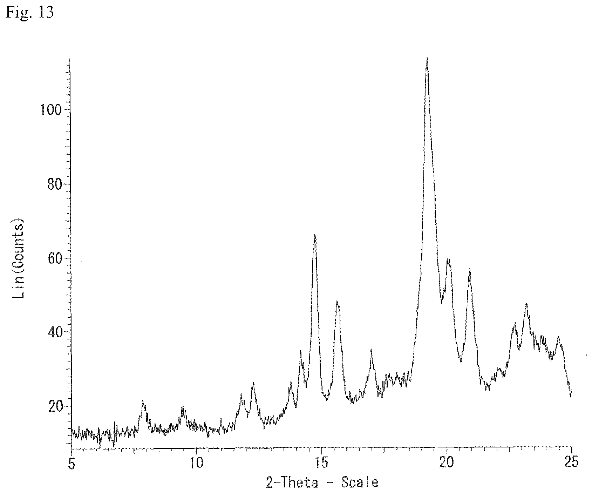

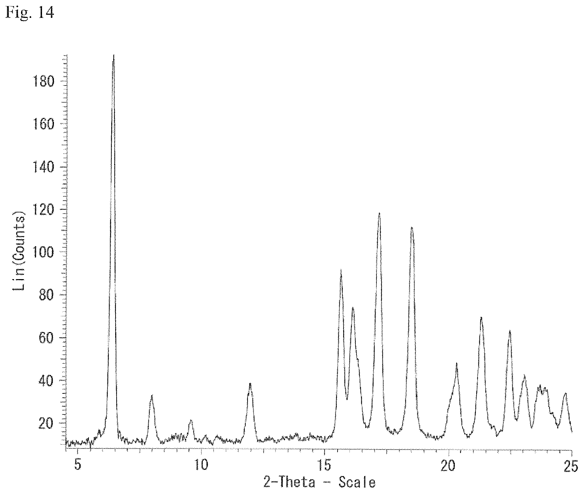

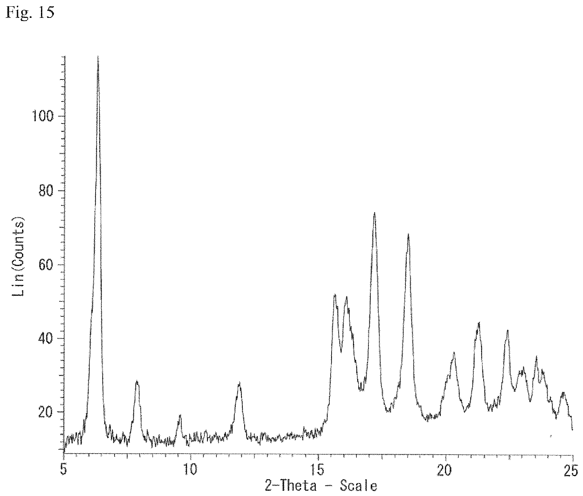

When the powder X-ray diffraction spectrum of monoacetate and ditosylate of Compound A were measured before and after storage, change in the spectra was recognized with monoacetate before and after storage (Figs. 3 and 13), whereas no significant peak change was recognized with ditosylate (Figs. 14 and 15). The spectrum of monoacetate after storage at 25°C-60% RH for 2 months was similar to the spectrum of pentahydrate of Compound A, suggesting that it was desalted.

### [Preparation Examples]

### Preparation Example 1:

### Tablet containing 5 mg of ditosylate of Compound A

The following ingredients are mixed in a conventional manner and compressed to give 10,000 tablets each containing 5 mg of an active ingredient.
- 4-({(4S)-1-(4-Carbamimidoylbenzoyl)-4-[4-(methylsulfonyl)piperazin-1-yl]-L-prolyl}amino) benzoic acid di(4-toluenesulfonate): 50 g
- Carboxymethyl cellulose calcium (disintegrant): 20 g
- Magnesium stearate (lubricant): 10 g
- Microcrystalline cellulose: 920 g

### Preparation Example 2:

### Injection preparation containing 20 mg of ditosylate of Compound A

After mixing the following ingredients in a conventional manner, the solution is sterilized in a conventional manner, and 5 mL aliquots are charged into ampules and lyophilized in a conventional manner to give 10,000 ampules each containing 20 mg of an active ingredient.
- 4-({(4S)-1-(4-Carbamimidoylbenzoyl)-4-[4-(methylsulfonyl)piperazin-1-yl]-L-prolyl}amino) benzoic acid di(4-toluenesulfonate): 200 g
- Mannitol: 20 g
- Distilled water: 50 L

### INDUSTRIAL APPLICABILITY

Ditosylate and dibcsylate of Compound A have extremely strong blood coagulation factor XIa inhibitory activity, and also are excellent in humidity stability and storage stability, thus are very useful as pharmaceutical ingredients.

## Claims

1. 4-({(4S)-1-(4-Carbamimidoylbenzoyl)-4-[4-(methylsulfonyl)piperazin-1-yl]-L-prolyl}amino) benzoic acid di(4-toluenesulfonate).

2. The 4-({(4S)-1-(4-Carbamimidoylbenzoyl)-4-[4-(methylsulfonyl)piperazin-1-yl]-L-prolyl}amino) benzoic acid di(4-toluenesulfonate) according to claim 2, which is in a crystal form.

3. The crystal according to claim 2, having, in a powder X-ray diffraction spectrum, at least two or more diffraction peaks at diffraction angles (20) selected from about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees.

4. The crystal according to claim 2, having, in a powder X-ray diffraction spectrum, diffraction peaks at diffraction angles (20) of about 6.2, about 7.8, about 9.4, about 11.7, about 15.6, about 16.0, about 16.3, about 17.1, about 18.6, about 20.0, about 20.3, about 21.3, about 22.4, about 23.0, about 23.5, about 23.8, about 24.1, about 24.7, about 25.9, and about 27.2 degrees.

5. The crystal according to claim 2, **characterized by** a powder X-ray diffraction spectrum chart shown in Fig. 1.

6. The crystal according to any one of claims 2 to 5, having an onset temperature of about 216°C or an endothermic peak temperature of about 228°C in differential scanning calorimetry.

7. The crystal according to claim 6, **characterized by** a differential scanning calorimetry chart shown in Fig. 5.

8. 4-({(4S)-1-(4-Carbamimidoylbenzoyl)-4-[4-(methylsulfonyl)piperazin-1-yl]-L-prolyl}amino) benzoic acid dibenzenesulfonate.

9. The 4-({(4S)-1-(4-Carbamimidoylbenzoyl)-4-[4-(methylsulfonyl)piperazin-1-yl]-L-prolyl}amino) benzoic acid dibenzenesulfonate according to claim 8, which is in a crystal form.

10. The crystal according to claim 9, having, in a powder X-ray diffraction spectrum, at least two or more diffraction peaks at diffraction angles (20) selected from about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees.

11. The crystal according to claim 9, having, in a powder X-ray diffraction spectrum, diffraction peaks at diffraction angles (20) of about 6.2, about 7.7, about 12.0, about 12.5, about 16.2, about 17.2, about 17.7, about 18.6, about 18.8, about 19.0, about 20.3, about 20.8, about 21.5, about 22.5, about 22.9, and about 23.1 degrees.

12. The crystal according to claim 9, **characterized by** a powder X-ray diffraction spectrum chart shown in Fig. 2.

13. The crystal according to any one of claims 9 to 12, having an onset temperature of about 203°C or an endothermic peak temperature of about 213°C in differential scanning calorimetry.

14. The crystal according to claim 13, **characterized by** a differential scanning calorimetry chart shown in Fig. 6.

15. A pharmaceutical composition comprising 4-({(4S)-1-(4-carbamimidoylbenzoyl)-4-[4-(methylsulfonyl)piperazin-1-yl]-L-prolyl}amino) benzoic acid di(4-toluenesulfonate) according to any one of claims 1 to 7.

16. A pharmaceutical composition comprising 4-({(4S)-1-(4-carbamimidoylbenzoyl)-4-[4-(methylsulfonyl)piperazin-1-yl]-L-prolyl}amino) benzoic acid dibenzenesulfonate according to any one of claims 8 to 14.

17. The pharmaceutical composition according to claim 15 or 16, which is a blood coagulation factor XIa inhibitor.

18. The pharmaceutical composition according to claim 15 or 16, which is a drug for preventing and/or treating blood coagulation factor XIa-related disease.

19. The pharmaceutical composition according to claim 18, wherein the blood coagulation factor XIa-related disease is thromboembolic disease.

20. The pharmaceutical composition according to claim 19, wherein the thromboembolic disease is acute coronary syndrome, disseminated intravascular coagulation (DIC) or cerebral infarction.
